(19) Europäisches Patentamt
European Patent Office
Office européen des brevets

(11) EP 1 851 316 B1

(12) **FASCICULE DE BREVET EUROPEEN**

(45) Date de publication et mention de la délivrance du brevet:
**09.04.2014 Bulletin 2014/15**

(21) Numéro de dépôt: **06709296.5**

(22) Date de dépôt: **10.02.2006**

(51) Int Cl.:
***C12N 15/79*** (2006.01) ***A61K 39/008*** (2006.01)

(86) Numéro de dépôt international:
**PCT/FR2006/000314**

(87) Numéro de publication internationale:
**WO 2006/085011 (17.08.2006 Gazette 2006/33)**

(54) **MOYENS POUR L'OBTENTION DE PROMASTIGOTES DE LEISHMANIES AVIRULENTS, PROMASTIGOTES OBTENUS ET LEURS APPLICATIONS**

MITTEL ZUR GEWINNUNG AVIRULENTER LEISHMANIA-PROMASTIGOTEN, GEWONNENE PROMASTIGOTEN UND ANWENDUNGEN DAVON

MEANS FOR OBTAINING AVIRULENT LEISHMANIA PROMASTIGOTES, PROMASTIGOTES OBTAINED, AND THE APPLICATIONS THEREOF

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **10.02.2005 FR 0501348**

(43) Date de publication de la demande:
**07.11.2007 Bulletin 2007/45**

(73) Titulaire: **Institut de Recherche pour le Développement ( IRD)**
**13572 Marseille Cédex 02 (FR)**

(72) Inventeurs:
- **LEMESRE, Jean-Loup**
  **F-34070 Montpellier (FR)**
- **HOLZMULLER, Philippe**
  **F-34560 Poussan (FR)**
- **BRAS-GONCALVES, Rachel**
  **F-34090 Montpellier (FR)**

(74) Mandataire: **Peaucelle, Chantal et al**
**Cabinet Armengaud Aîné**
**3, Avenue Bugeaud**
**75116 Paris (FR)**

(56) Documents cités:
- **CHEN D Q, ET AL.: "EPISOMAL EXPRESSION OF SPECIFIC SENSE AND ANTISENSE MRNAS IN LEISHMANIA AMAZONENSIS: MODULATION OF GP63 LEVEL IN PROMATIGOTES AND THEIR INFECTION OF MACROPHAGES IN VITRO" INFECTION AND IMMUNITY, vol. 68, no. 1, 2000, - 1980 page 86, XP009051114**
- **HAJMOVA M, ET AL.: "DOWN REGULATION OF GP63 IN LEISHMANIA AMAZONENSIS REDUCES ITS EARLY DEVELOPMENT IN LUTZOMYIA LONGIALPIS" MICROBES AND INFECTION, vol. 6, 2004, pages 646-649, XP009051117**
- **JOSHI P B, ET AL.: "TARGETED GENE DELETION IN LEISHMANIA MAJOR IDENTIFIES LEISHMANOLYSIN (GP63) AS A VIRULENCE FACTOR" MOLECULAR AND BIOCHEMISCAL PARASITOLOGY, vol. 120, 2002, pages 33-40, XP009051113**
- **BEVERLEY S M AND DOBSON D E: "FLYPAPER FOR PARASITES" CELL, vol. 119, 29 octobre 2004 (2004-10-29), pages 311-316, XP009051110**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 851 316 B1

## Description

L'invention a pour objet des moyens pour l'obtention de promastigotes de leishmanies avirulents. Elle vise également les promastigotes obtenus et leurs applications, en particulier en immunopropylaxie.

Les leishmanioses constituent des infections parasitaires endémiques à répartition mondiale qui représentent un problème important de santé publique affectant aussi bien des pays en voie de développement, que des pays tels que ceux de l'Europe du Sud, où elles sont considérées comme des maladies opportunistes chez les sujets immunodéprimés.

A l'heure actuelle, il n'existe pas de moyens immunoprophylactiques, ni immunothérapeutiques, efficaces contre les leishmanioses, et plus particulièrement contre l'agent infectieux responsable, à savoir les leishmanies.

Les leishmanies sont des protozoaires flagellés appartenant à la famille des *Trypanosomatidae* et au genre *Leishmania.* Cette famille inclut des parasites qui peuvent infecter l'homme, et certains animaux comme les rongeurs, les canidés, ou les mammifères.

Le cycle biologique des protozoaires du genre *Leishmania* se partage entre un hôte vecteur invertébré, le phlébotome (insecte diptère hématophage de petite taille), et de nombreux vertébrés, dont l'homme. Les leishmanies possèdent un cycle de développement parasitaire hétéroxène au cours duquel se succèdent deux stades morphologiques distincts. Chez le phlébotome, le parasite se présente sous une forme flagellée, extracellulaire, mobile à l'intérieur du tube digestif de l'insecte, appelée stade promastigote. Chez l'hôte invertébré, le parasite se transforme en une forme ovoïde de 2 à 6 μm de diamètre , immobile, avec un résidu flagellaire très court, appelée amastigote, qui va résider à l'intérieur de la vacuole parasitophore, au niveau des cellules du système réticulo-histiocytaire, principalement des macrophages.

L'insecte vecteur hématophage femelle s'infecte par le sang de l'hôte vertébré contaminé, par ingestion de macrophages parasités. La plupart des amastigotes ingérés sont alors détruits dans les heures qui suivent. Les survivants se transforment en promastigotes, qui vont coloniser l'intestin médian où ils se multiplient activement. Ils migrent ensuite vers les parties antérieures et buccales du tractus digestif et subissent alors une une différenciation conduisant à la formation de promastigotes métacycliques très infectieux pour l'hôte vertébré.

L'hôte vertébré est contaminé par les promastigotes métacycliques lors d'une piqûre par un phlébotome femelle infecté. Ces formes promastigotes sont injectées dans le derme conjointement à la salive, puis vont être phagocytées par les cellules du sytème réticulo-hystiocytaire, notamment des macrophages, à l'intérieur desquelles elles se différencient en formes amastigotes intracellulaires immobiles. Elles vont alors se multiplier activement par scissiparités au sein de la vacuole parasitophore (phagolysosome) jusqu'à provoquer la lyse de la cellule-hôte, permettant ainsi aux amastigotes libérés de propager l'infection en colonisant d'autres macrophages sains. Une nouvelle piqûre par un phlébotome va provoquer l'infection de celui-ci et permettre au parasite de perpétuer son cycle.

Malgré les avancées dans ce domaine, les leishmanioses demeurent encore aujourd'hui un grave problème de santé publique en raison du nombre de personnes concernées, de l'étendue de la zone géographique couverte par la maladie, ainsi que de la diversité et de la gravité des tableaux cliniques observés.

On mesure donc le besoin de vaccins efficaces contre les leishmanioses et de nouvelles cibles thérapeutiques et/ou vaccinales pour traiter ces maladies parasitaires.

Lors de l'infection, les formes promastigotes infectieuses injectées à l'hôte mammifère doivent échapper à la réponse immunitaire de l'hôte afin de pouvoir envahir par la suite les macrophages. Le parasite doit mettre alors en oeuvre des mécanismes lui permettant d'éviter cette réponse immunitaire et être capable de s'adapter à un nouvel environnement. La reconnaissance précoce du parasite par l'organisme hôte lors de l'invasion peut être ainsi une étape clé de la résistance à l'infection.

Des antigènes de surface, le lipophosphoglycane (LPG) et la gp63 (métalloprotéase) encore appelée leishmanolysine, ont été découverts dans les années 1980.

Le LPG est la molécule majeure de surface des formes promastigotes dont le rôle en tant que facteur de virulence a été confirmé.

La gp63 est une protéase majeure de surface, également considérée comme un facteur de virulence.Cette protéine est décrite par Chen et al, dans Infection and Immunity, 200, p80-86 et Hajmovà et al, dans Microbes and Infection 6 (20.04) p646-649.

Ces auteurs ont étudié l'expression de cette protéine et sa participation dans une infection par *L. amazonensis.*

Un troisième type d'antigènes, dénommé PSA pour « Promastigotes Surface Antigens » a été découvert en 1988-1989. Ils constituent les antigènes majeurs de surface des formes promastigotes. Ainsi, Lohman et al, dans P.N.A.S. USA, vol 87, p 8399-8397, 1990, décrivent une protéine de surface de *L. amazonensis,* gp 46/M-2.

Les antigènes d'excrétion-sécrétion (AES), très étudiés chez de nombreux microorganismes parasites (nématodes,

protozoaires...), jouent un rôle important dans les mécanismes d'infection et de défense de ces parasites, puisqu'ils ont la possibilité d'agir à distance. Chez les leishmanies, les travaux de recherche ont longtemps été limités par la difficulté de pouvoir disposer d'AES en quantités suffisantes pour les étudier.

Tous ces antigènes semblent jouer un rôle déterminant dans la survie et l'infectivité des formes promastigotes.

Les travaux antérieurs de l'inventeur dans ce domaine avaient conduit à l'élaboration de milieux de culture complètement définis, sans macromolécule et dépourvus de sérum de veau foetal, permettant la culture en continue des formes promastigotes et amastigotes en conditions axénique et asérique. Ces milieux décrits notamment dans la demande de brevet FR n° 93 05 779 ont permis de reproduire *in vitro* le cycle complet de développement parasitaire de leishmanies, telles que *L. amazonensis* et *L. infantum,* et de disposer, dans des conditions naturelles, d'une source relativement abondante d'AES par une simple concentration du surnageant de milieu de culture métabolisé par les parasites. Une caractérisation des AES de leishmanies a ainsi pu être réalisée.

Des études menées au laboratoire des inventeurs ont ainsi montré que l'immunogène majeur des AES de *L. amazonensis* avait une masse moléculaire de 45 kDa. Le criblage d'une banque d'expression d'ADNc de formes promastigotes par un anticorps monoclonal appelé E2 produit contre cet immunogène majeur a permis d'isoler et d'identifier des clones d'ADNc codant des protéines de la famille des PSA. Cet anticorps est produit par l'hybridome murin IV D6/E2 déposé à la C.N.C.M, 28 rue du Dr Roux, Paris, France, le 10 novembre 2004, sous le n°I-3318, plus spécialement par l'hybridome murin déposé le 3 février 2006 à la CNCM sous le n°I-3570. C'est ainsi que pour la première fois trois nouvelles séquences d'ADNc de PSA exprimées chez la forme promastigote de *L. amazonensis* ont été identifiées. Ces ADNc isolés diffèrent les uns des autres par le nombre de LRR (4, 6 ou 7). Plus récemment, un criblage d'une banque cosmidique de promastigotes de *L. infantum* a été réalisé en utilisant comme sonde radiomarquée l'ADN codant pour la PSA de *L. amazonensis*. Un nouveau gène de *Leishmania, IJ11,* a été identifié, codant une protéine excrétée/sécrétée de *L. infantum,* de masse moléculaire de 56 kDa (LiPSA 50s), appartenant à la famille des PSA.

Une étude fonctionnelle des gènes codant pour des PSA, immunogènes majeurs des produits d'excrétion/sécrétion des leishmanies, a donc été entreprise en utilisant les outils offerts par la génomique. L'approche expérimentale a consisté à introduire dans les promastigotes d'une leishmanie des constructions plasmidiques susceptibles d'induire une diminution du niveau de production de la PSA afin d'étudier les répercussions de ces changements sur la biologie du parasite et sa virulence.

Il est ainsi apparu que le clonage non directionnel dans un vecteur d'expression du gène *IJ11* codant la protéine LiPSA 50s conduisait, après sélection, à l'obtention de plasmides contenant l'insert dans une orientation antisens (Ldi antisens) permettant la sous-expression de la PSA chez les promastigotes modifiés de *L.infantum.*

De manière avantageuse, ces résultats se sont avérés généralisables, aussi bien en ce qui concerne les espèces de leishmanies que les inserts de gènes codant pour des protéines antigéniques utilisables.

L'invention a donc pour but de fournir de nouvelles constructions de vecteurs d'expression de gènes codant pour des protéines antigéniques de promastigotes de leishmanies.

Elle vise également l'utilisation de ces constructions pour obtenir des mutants de promastigotes de leishmanies avirulents.

Selon encore un autre aspect, l'invention vise les applications de ces mutants avirulents à des fins immunoprophylactiques.

Un vecteur d'expression selon l'invention est caractérisé en ce qu'il renferme un insert, dans une orientation antisens, d'un gène codant pour un antigène d'excrétion/sécrétion.

De manière avantageuse, le clonage dans un vecteur d'expression dudit gène dans une orientation antisens permet la sous-expression du peptide ou de la protéine dans les promastigotes modifiés de leishmanies.

De manière préférée, ledit vecteur est caractérisé en ce qu'il renferme un insert de gène de *L. amazonensis.*

Le gène de PSA de *L.amazonensis* répond plus spécialement à l'une des séquences suivantes : SEQ ID N°1 à 5.

Dans un autre mode de réalisation préféré de l'invention, il s'agit d'un insert de gène de *L.infantum.*

Le gène de *L.infantum* est avantageusement caractérisé en ce qu'il répond à la SEQ ID N°6.

L'invention vise également les promastigotes de leishmanies génétiquement modifiés, caractérisés en ce qu'il s'agit de mutants avirulents, transfectés par un vecteur tel que défini ci-dessus.

L'invention vise en outre un procédé d'obtention de formes avirulentes de promastigotes de leishmanies, caractérisé en ce qu'il comprend

- l'insertion de manière non directionnelle dans un vecteur d'expression de gènes codant pour un antigène d'excrétion/sécrétion,
- la sélection des vecteurs renfermant les inserts dans une orientation antisens,
- la transfection de formes promastigotes de leishmanies à l'aide des vecteurs sélectionnés.
- le clonage biologique par micromanipulation des promastigotes génétiquement transformés, pour l'obtention des mutants sous-exprimant, voire n'exprimant plus, le gène codant une protéine antigénique de promastigote de leishmanie.

La transfection des formes promastigotes est avantageusement réalisée par électroporation.

La révélation par Western blot du peptide ou de la protéine permet d'estimer son niveau de production chez les clones.

La mise en oeuvre des dispositions qui précèdent permet d'étudier les répercussions de la sous-expression de peptides ou protéines antigéniques de leishmanies sur le comportement des promastigotes transformés et d'identifier ainsi leur rôle biologique.

A cet effet, les différents parasites génétiquement modifiés ont été clonés par micromanipulation (clonage biologique) et caractérisés et comparés au niveau moléculaire (niveau de production de la protéine antigénique, niveau d'expression des transcrits correspondants). La caractérisation phénotypique des promastigotes génétiquement modifiés et clonés a consisté à comparer les cinétiques de croissance de ces parasites ainsi que leur pouvoir infectieux *in vitro* vis-à-vis de macrophages canins.

La caractérisation d'un facteur de virulence parasitaire, tel que la PSA, permet de développer de nouvelles stratégies d'interférence avec le processus infectieux à des fins thérapeutiques et/ou vaccinales.

En particulier, l'invention permet d'obtenir des souches atténuées, voire avirulentes, utilisables en vaccination animale et/ou humaine.

D'autres caractéristiques et avantages de l'invention sont donnés dans les exemples qui suivent avec référence aux figures 1 à 4, qui représentent, respectivement,

- les figures 1 et 2, les résultats d'analyse par Western blot de la PSA d'extraits polypeptidiques totaux d'une souche sauvage de Ldi Mon1 et d'antigènes d'excrétion-sécrétion de surnageants de culture de souches sauvage, de souches mères de parasites transformés sens et antisens et de leurs clones;
- la figure 3, des résultats de RT-PCR sur des transcrits de gène codant la PSA et de gène de ménage;
- la figure 4, l'indice parasitaire déterminé au cours de l'infection *in vitro* de macrophages canins par une souche sauvage ou des clones.

## MATERIEL ET METHODE

### I- Cultures *in vitro*

1- Cultures axéniques de *L. infantum*

La souche sauvage de *Leishmania infantum* utilisée dans les études décrites ci-après porte la référence O.M.S.: MHOM/MA/67/ITMAP-263/clone2, zymodème MON-1. Les souches transformées ont été obtenues après clonage du gènes IJ11 (1404pb), codant la PSA de *L. infantum* (56kDa), en position sens ou antisens dans le vecteur d'expression pTEX, puis transfection par électroporation chez la forme promastigote de *L. infantum.* Les parasites transformés Ldi pTEX, Ldi- IJ11 antisens ont été sélectionnés en présence de concentrations croissantes de généticine jusqu'à atteindre 50μg/ml. La présence du plasmide et l'orientation du transgène (antisens) ont été vérifiées par Southern Blot et le niveau de transcription du transgène par Northern Blot.

Les formes promastigotes sont cultivées en condition axénique dans un milieu de culture complètement défini ne contenant aucune macromolécule en tant que substitut sérique (milieu asérique RPMI 199H pH 7), mais également en milieu RPMI 1640 contenant 20% de sérum de veau foetal décomplémenté (milieu sérique RPMI 20% pH 7.2), et additionné de pénicilline (100 unités/ml) et de streptomycine (100μg/ml). Les différentes souches transformées ont été clonées par la technique de micromanipulation. Un parasite unique est prélevé sous microscope à l'aide d'une fine aiguille de verre et déposé sur milieu NNN (Novy, macNeal, Nicolle). La culture des parasites est réalisée par repiquages successifs en milieu RPMI 20%. Les formes promastigotes génétiquement modifiées ainsi que les clones correspondants sont maintenus en culture continue par repiquages hebdomadaires dans des milieux de culture sérique ou adaptés en milieu asérique à 25°C en présence de généticine (50μg/ml). La concentration parasitaire est déterminée par cytométrie de flux (FacsCalibur, Beckton Dickinson). La conservation des parasites est assurée par cryocongélation des organismes en culture en présence de 3% DMSO (Diméthylsulfoxide) à -180°C dans l'azote liquide.

2- Cultures de macrophages

Les PBMC (Peripheral Blood Mononuclear Cells) sont isolés de sang total de chien sain par gradient de Ficoll (Ficoll Lymphoprep®, Density 1.077±0.001g/ml; AbCys, France) et par centrifugation à 700g pendant 20 min à température ambiante. Les cellules sont ensuite lavées 3 fois dans du PBS (Phosphate Buffer Saline 0.01M pH 7.2) sans $Ca^{2+}/Mg^{2+}$ par centrifugation à 400g pendant 10 min à 4°C, avant d'être resuspendues dans du RPMI 1640 complémenté par 10% de sérum de veau foetal, 100 unités/ml de pénicilline et 100µg/ml de streptomycine, puis cultivées à 37°C.

**II- Récolte et préparation du matériel parasitaire**

1- Récolte du matériel parasitaire

Les formes promastigotes en phase stationnaire des croissance (7 jours) sont récoltées par centrifugation des cultures parasitaires asériques à 3000g pendant 10 min à 4°C. Les culots parasitaires et les surnageants de culture ainsi dissociés sont ensuite traités séparément.

2- Préparation de lysats parasitaires

Le culot parasitaire est lavé 2 fois en PBS dans les conditions de centrifugation décrites précédemment. Après le dernier lavage, le nombre de parasites récoltés est déterminé par cytométrie de flux. $10^9$ parasites lavés sont remis en suspension dans une solution de lyse hypotonique : Tris 50mM pH 8 contenant 1% de Triton X100 (Sigma). Après 20 min d'incubation à 4°C, une centrifugation de 20 min à 4°C et à 2000g permet de séparer le matériel insoluble (débris) du surnageant (molécules solubles). Ce surnageant correspond à un extrait polypeptidique total (EPT).

3 - Concentration des antigènes d'excrétion-sécrétion (AES) à partir du surnageant de culture métabolisé par lest parasites

10ml de surnageant métabolisé par les parasites sont filtrés sur membrane Millipore, (0,22 µm), puis congelés en coquille avant d'être lyophilisés. Les lyophilisats sont repris dans 1ml d'eau milliQ pour être dialysés 48 h à 4°C de façon à éliminer les sels. Une seconde lyophilisation permet de concentrer le surnageant 200 fois.

**III- Analyses biochimiques**

1- Dosage protéique

Le dosage des protéines est réalisé à l'aide du spectrophotomètre Beckman DU-600, à 595 nm selon la méthode de Bradford, en utilisant le réactif Biorad (Biorad Protein Assay Kit 11). Une gamme étalon, de 5 à 20µg/ml, est préparée à, partir d'une solution standard d'albumine bovine (BSA 1mg/ml, Sigma).

2- Analyse électrophorétique en gel de polyacrylamide SDS-PAGE (Sodium Dodecyl Sulfate-Polyacrylamide Gel Electrophoresis)

L'analyse en gel de polyacrylamide SDS-PAGE a été réalisée dans une cuve d'électrophorèse de type minigel (Biorad). Les pourcentages d'acrylamide utilisés sont de 5% pour le gel de concentration et de 10% pour le gel de séparation, ce qui permet de séparer les protéines de 10 à 200kDa. Des concentrations protéiques d'EPT sont mélangées (v/v) avec du tampon échantillon contenant 4% de SDS, 20% de glycérol et 0,5% de bleu de Bromophénol dans du Tris-HCl 0,5M pH 6,8.

La migration électrophorétique s'effectue dans un tampon (Tris 2, 5mM/Glycine 19,2mM pH 8,3) contenant 0,1% de SDS sous un voltage constant de 80V. L'étalonnage du gel est obtenu par le dépôt de 10 µg de protéines de poids moléculaires connus, standards (Mark12 Wide Range Protein Standard/SeeBlue® Plus2 Pre-Stained Standard, Novex).

3- Révélation des protéines au nitrate d'argent

Après élimination du SDS par lavage en présence d'acide acétique (10%) et d'éthanol (40%) pendant 1h, l'acide acétique est ensuite lui-même éliminé par deux rinçages de 30 min dans 50% d'éthanol. Puis les gels sont mis à incuber pendant 15 min dans un mélange de coloration: $AgNO_3$ 19,4% - NaOH 0,36%. Après 5 lavages dans l'eau milliQ, une solution de révélation contenant du formaldéhyde à 0,02% dans de l'acide citrique à 1% (p/v), fait apparaître sur le gel les protéine. Le seuil de révélation de cette méthode de coloration est de 2 ng équivalent protéine.

**IV- Analyses immunologiques**

1- Western-Blot

Après avoir été séparées en gel de polyacrylamide SDS-PAGE, les protéines sont transférées sur membrane de nitrocellulose (Amersham, Hybond-C Extra). Le transfert se fait en condition semi-sèche. Les membranes sont ensuite saturées une heure dans une solution bloquante TBS-lait 5%. Les membranes sont incubées la nuit, à température ambiante et sous agitation, avec l'anticorps monoclonal F5 dirigé contre la PSA et obtenu chez la souris (AMC F5) dilué au $1/50^{e}$ dans du TBS. Après 3 lavages de 5 min dans du TBS, les anticorps fixés sont détectés par des anticorps secondaires anti-immunoglobuline G de souris couplés à la peroxydase (Tebu) dilués au $1/500^{e}$ dans du TBS et incubés pendant 45 min, à température ambiante sous agitation. Ensuite, 3 lavages de 5 min dans du TBS sont réalisés avant de procéder à la révélation de la réaction par une solution contenant 15mg de 4-chloro-1-naphtol dissout dans 5ml de méthanol, auquel sont ajoutés 25ml de TBS contenant 15μl de $H_2O_2$ 30%. La réaction est stoppée par lavage de la membrane à l'eau distillée.

2- Immunofluorescence indirecte sur des parasites

Les parasites en phase stationnaire de leur culture (7 jours) sont fixés avec du formaldéhyde à 1%, dans du PBS sans $Ca^{2+}/Mg^{2+}$, puis lavés. Une fois fixés, les parasites sont déposés sur les puits téflonés (10 puits, 50ml/puit) et séchés à l'air libre. Chaque puit est alors traité avec l'anticorps monoclonal F5 (AMC F5) dilué dans une solution bloquante (de saturation) (1% de sérum de chèvre, 0.5% de BSA dans du PBS) pendant 30 min à 37°C. Les cellules sont ensuite lavées et incubées pendant 30 min à 37°C avec le second anticorps conjugué à l'isothiocyanate de fluorescéine (FITC) (antisérum de chèvre anti-immunoglobuline G de souris) dilué au $1/300^{e}$ dans une solution de PBS contenant du bleu Evans au 1/7500. Les lames sont observées au microscope photonique à fluorescence (Leitz).

**V- Analyse moléculaire**

1- Extraction des ARN totaux

Les formes promastigotes sont récoltées par centrifugation des cultures parasitaires asériques à 3000g pendant 10 min à 4°C. Le culot parasitaire est lavé 2 fois en PBS dans les conditions de centrifugation précédentes. Après le dernier lavage, afin de lyser les parasites et d'inhiber les ribonucléases (RNAse) endogènes, les cellules sont dissociées par vortex dans 1ml de Trizol (Trizol Reagent, GibcoBRL), puis incubées 5 min à température ambiante. Le lysat est incubé dans 0,2ml de chloroforme pendant 2 min à température ambiante, puis centrifugé à 12000g pendant 15 min à 4°C pour donner deux phases, aqueuse et organique. Les ARN se trouvent exclusivement dans la phase aqueuse qui est récupérée. Les ARN sont alors précipités par addition de 0,5ml d'isopropanol et incubés pendant 10 min à température ambiante, puis centrifugés à 12000g pendant 10 min à 4°C. Le culot est lavé dans lml d'éthanol à 75%, puis centrifugé à 7500g pendant 5 min à 4°C, avant d'être repris dans 40 μl d'eau (RNAse free). Afin d'éliminer une présence éventuelle d'ADN, le culot d'ARN dissout est repris dans 1μl de DNAse I + 0.1 volume de tampon DNAse I 10X et incubé pendant 20 min à 37°C. Après addition de 5μl de DNAse Inactivation Reagent, incubation pendant 2 min à température ambiante, puis centrifugation à 10000g pendant 1 min à 4°C, le surnageant contenant les ARN est récupéré. L'ARN total est quantifié par spectrophotométrie par lecture d'absorbance à 260nm (1 unité DO = 40μg/ml d'ARN simple brin). La qualité de l'ARN extrait, la quantité et la taille sont vérifiées par électrophorèse sur gel d'agarose à 1% en tampon TAE 0.5X, en présence de bromure d'éthidium (BET) à 0.01% Le marqueur de taille utilisé est le SmartLadder (Eurogentec). L'ARN total est alors visualisé sous UV à 312nm après migration. Les critères de qualité de l'extraction retenus sont l'absence d'ADN et la visualisation de 3 bandes d'ARN ribosomaux d'intensité égale.

2- RT-PCR

L'ADNc simple brin est synthéthisé en réalisant une RT-PCR avec 1μg d'ARN total, 2μl de dNTP mix (5 mM), 0.2μl d'amorces spécifiques (10 μM) et $H_2O$ qsp 13 μl. Le mix est incubé pendant 2 min à 65°C puis 2 min dans la glace avant que soient additionnés 4 μl de Buffer 5X, 1 μl de DTT (0,1M), 1 μl de Superase In (40U) (RNAse Inhibitor, Ambion) et 1 μl de SuperScript™ II (200U) (Reverse Transcriptase, Invitrogen). Le mix est incubé pendant 50 min à 42°C, puis la réaction est inactivée par choc thermique pendant 15 min à 70°C.

L'ADNc simple brin est amplifié par PCR avec 3 μl d'ADNc, 12,5 μl de PCR Mastermix (Promega), 1 μl d'amorces spécifiques 5' P et 3' P (10 μM) et $H_2O$ qsp 25 μl.

Les amorces suivantes ont été utilisée :

SEQ ID N°7 - nhinspl : 5'-CATCGCTTACCCCTTGTT-3'
SEQ ID N°8 - nhinsp2 : 5'-GTGGCCAATCGCGACA-3'
SEQ ID N°9 - PIRT : 5'-ATGGCGCTGTGCGTGCGTCGG-3'
SEQ ID N°10 - P4RT : 5'-GCGCGCGACAGCAGCGGCAT-3.'

Le programme de la réaction de PCR utilisé et réalisé dans un thermocycleur (GeneAmp PCR System 2400, Applied Biosystems) est le suivant : 5 min à 94°C, puis 27 cycles (94°C 30 sec, 55°C 1 min, 72°C 1 min) et 10 min à 72°C.
Les produits PCR sont vérifiés par électrophorèse sur gel d'agarose 1%.

**VI- Analyse biologique**

1- Cinétique de croissance

Les cinétiques de culture *in vitro* ont été réalisées sur 10 jours en milieu RPMI 2.0%, en partant d'une concentration initiale de 5.105 parasites/ml. La concentration parasitaire est déterminée par cytométrie de flux (FacsCalibur, Beckton Dickinson) en mélangeant 500μl de PBS, 5μl de culture et 0.5μl d'iodure de propidium.

2- Infection *in vitro* dans des interactions parasites / macrophages

$10^6$ PBMC/puits (200μl), isolés de sang total de chien sain, sont distribués dans des chambres de culture LabTek® (Nalge Nunc International). Après 30 min d'incubation à 37°C 5% $CO_2$ les LabTek® sont lavés doucement avec du RPMI 10% pour éliminer les cellules qui n'ont pas adhéré. Les macrophages sont obtenus à partir des monocytes de culture après 5 jours de maturation. Les macrophages matures sont infectés *in vitro* avec des formes promastigotes de phase stationnaire de culture pendant 30 min, 2 h et 48 h à 37°C et 5% $CO_2$ à un ratio parasites:macrophage de 10:1. Les macrophages sont alors lavés doucement 2 fois pour éliminer les parasites non-phagocytés. Après 30 min, 2 h et 48 h, les cellules sont fixées au méthanol et colorées au Giemsa. 300 macrophages sont observés au microscope optique à immersion (grossissement 1000X), et le pourcentage de macrophages parasités, le nombre d'amastigotes par macrophages, ainsi que l'index parasitaire (I.P.) sont déterminés. L'I.P. équivaut au pourcentage de macrophages infectés multiplié par le nombre d'amastigotes par macrophages.

RESULTATS

-Evaluation du niveau de production de la PSA chez les clones transformés

On rapporte sur les figures 1 et 2 les résultats d'analyse par Western blot de la PSA, respectivement,

- des extraits polypeptidiques totaux (EPT) issus des culots parasitaires de la souche sauvage Ldi Monl,
- sur des anitgènes d'excrétion-sécrétion (AES) issus de surnageants de milieu de culture métabolisés, de la souche sauvage Ldi Monl, des souches mères de parasites transformés (Ldi pTEX, Ldi IJ11 sens et Ldi IJ11 antisens et de leurs clones correspondants (5μg équivalent protéine d'extrait total par piste). On constate qu'aucune bande n'est observée pour la souche sauvage Ldi Monl, que la surexpression est en revanche bien visible pour Ldi IJ11 sens aussi bien au niveau des EPT que des AES, comparativement à l'expression chez Ldi pTEX, et qu'une diminution de l'expression de la PSA est observée avec Ldi IJ11 antisens.

- Analyse des transcrits de la PSA par RT-PCR

On a réalisé une RT-PCR sur les transcrits du gène codant la PSA ainsi que sur les transcrits d'un gène de ménage codant la nucléoside hydrolase.
Les résultats obtenus sont donnés sur la figure 3. On constate une augmentation significative du niveau des transcrits codant la PSA chez Ldi IJ11 sens et une diminution notable chez Ldi IJ11 anti, comparativement à Ldi pTEX. Aucun signal n'est observé avec le souche sauvage Ldi Monl.

-Pouvoir infectieux *in vitro* dans les interactions parasites-macrophages

Sur la figure 4, on rapporte l'indice parasitaire déterminé au cours de l'infection *in vitro* de macrophages canins par la souche sauvage ou les différents clone sélectionnés à différents temps d'incubation : 30 min (attachement des parasites aux macrophages), 2 h (pénétration des parasites) et 48 h (survie et multiplication des amastigotes.
On constate qu'à 30 min et à 2h, les promastigotes Ldi IJ11 sens présentent un indice parasitaire environ deux fois plus important que ceux de Ldi lj11 antisens est environ deux fois plus faible. Les mêmes résultats sont obtenus après 48 h d'infection.

**VII -Etude du rôle in vivo de mutant pTEX, Sens et Antisens**

Infection de souris Balb/C par IP avec $10^8$promastigotes/100µl Protocole de mesure de la Parasitémie (Parasite burdens)

<u>Réf biblio :</u> Buffet P.A. et al. 1995. Antimicrobial agents and chemotherapy. Vol 39:2167-2168

<u>Préparer :</u> 1 tube de 15ml <u>par organe</u> avec 4ml de milieu SDM ou RPMI/20%SVF
Peser les tubes + milieu (poids= T en g) Cell Strainer + petites boîtes de pétri (1 cell strainer pour deux même organes appartenant au même groupe/lot de souris) + seringue de 1ml

Sur organes rate et foie :

Pour la rate : prélever toute la rate Pour le foie : ne prélever que le petit lobe

- Peser des organes (organe dans tube + milieu, poids= TO en g)

- Broyage/homogénéisation par potter ou cell-strainer dans 4 ml de milieu

Pour Parasitémie/Rate :

- En plaque 96 puits, prélever 250µl de cellules pour le 1ier puits et faire des dilutions de 2 en 2 avec 150µl de parasites dans 150µl de milieu, puis pour les puits suivants préléver 150µl à diluer dans 150µl de milieu...Faire 2 x 12 puits de dilutions (2 rangées)
- Après 7 et 15 jours d'incubation à 26-28°C, les plaques sont examinées au microscope inversé (objectif x100 ou x200)
- Noter l'absence ou la présence de parasite pour chaque puits. Noter le dernier puits où il y a présence parasites.

Pour Parasitémie/Foie :

- En plaque 96 puits, prélever 25µl de cellules pour le 1ier puits contenant 225µl de milieu et faire des dilutions de 4 en 4 avec 50µl de parasites dans 150µl de milieu, puis pour les puits suivants préléver 50µl à diluer dans 150µl de milieu...Faire 2 x 12 puits de dilutions (2 rangées)
- Après 7 et 15 jours d'incubation à 26-28°C, les plaques sont examinées au microscope inversé (objectif x100 ou x200)
- Noter l'absence ou la présence de parasite pour chaque puits. Noter le dernier puits où il y a présence parasites.

Le titre final correspond à la dernière dilution pour laquelle le puits contient au moins 1 parasite.
Le nombre de parasite par gramme (parasite burden) dans l'organe correspondant est calculé de la façon suivante :

Nombre de parasites = (moyenne géométrique des titres inverses de chaque duplicata /poids de coupe homogénéisée) x 400

$$\text{Charge parasitaire} = \frac{\text{Inverse de la dilution limite}}{\text{Poids organe (g)}} \times 400$$

N.B. : - 400 correspond à la fraction inverse de l'organe homogénéisée inoculée dans le 1er puit

$< 10^3$ à $>10^6$

Point à 14 jours après infection:
Parasitémie (lecture à 15 jours) réalisée en duplicata et dilutions de 2 en 2

$$\text{Charge parasitaire} = (\text{Inverse de la dilution limite/Poids organe (g)}) \times 400$$

| | Poids organe (g) | Dilution limite | Charge parasitaire parasite g-1 | Moyenne Charge parasitaire | Ecart type | Moyenne générale Charge parasitaire | Ecart type g | |
|---|---|---|---|---|---|---|---|---|
| Lot 1 pTEX | | | | | | | | |
| Souris 1 rate | 0,14 | 0,0078 | 366300 | 274725 | 129507 | 107912 | 56756 | sans souris 1 essai |
| | 0,14 | 0,0156 | 183150 | 183150 | | | | |
| Souris 2 rate | 0,16 | 0,0156 | 160256 | 120064 | 56840 | | | |
| | | | | | | | | sans souris 1 essai |
| | 0,16 | 0, 0313 | 79872 | | | 67348 | 22927 | 1 et 2 et |
| Souris 3 rate | 0,14 | 0,0625 | 45714 | 68498 | 32222 | | | sans souris 2 essai |
| | 0,14 | 0,0313 | 91283 | | | | | |
| souris 4 rate | 0,16 | 0,0625 | 40000 | 59936 | 28194 | | | |
| | 0,16 | 0,0313 | 79872 | | | | | |
| | | | | | | résultats en rouge non pris en compte | | |
| Lot 2 Sens | | | | | | | | |
| Souris 1 rate | 0,14 | 0,5 | 5714 | 2857 | 4041 | 2367 | 1517 | |
| | 0,14 | rien | 0 | | | | | |
| souris 2 rate | 0,19 | rien | 0 | 2105 | 2977 | | | |
| | 0,19 | 0, 5 | 4211 | | | | | |
| souris 3 rate | 0,24 | 1 | 1667 | 833 | 1179 | | | |
| | 0,24 | rien | 0 | | | | | |
| souris 4 rate | 0,17 | 0,25 | 9412 | 7059 | 3328 | | | |
| | 0,17 | 0,5 | 4706 | 4706 | | | | |
| souris 5 rate | 0,15 | 1 | 2667 | 1333 | 1886 | | | |
| | 0,15 | rien | 0 | | | | | |

(suite)

résultats en rouge non pris en compte

| | | | | | | | | | |
|---|---|---|---|---|---|---|---|---|---|
| Lot 2 Sens | | | | | | | | | |
| Lot 3 AntiSens | | | | | | | | | |
| Souris 1 rate | | 0,13 | 0,25 | 12308 | 12308 | 0 | 8744 | 2458 | |
| | | 0,13 | 0,25 | 12308 | | | | | |
| souris 2 rate | | 0,14 | 1 | 2857 | 1429 | 2020 | | | |
| | | 0,14 | rien | 0 | | | | | |
| souris 3 rate | | 0,15 | 0,5 | 5333 | 8000 | 3771 | | | |
| | | 0,15 | 0,25 | 10667 | | | | | |
| souris 4 rate | | 0,18 | 0,25 | 8889 | 6667 | 3143 | | | |
| | | 0,18 | 0,5 | 4444 | | | | | |
| souris 5 rate | | 0,20 | 0,25 | 8000 | 8000 | 0 | | | |
| | | 0,20 | 0,25 | 8000 | | | | | |
| Récapitulatif Point 15 j | | | | | | | | | |
| | Moyenne Charge parasitaire | Ecart type | Ecart Moyen | | | | | Moyenne Charge parasitaire | Ecart type Ec |
| Lot 1 pTEX | | | | | | | pTEX souris 2 rate | 120064 | 56840 |
| Souris 1 rate | | 274725 | 129507 | 91575 | | | pTEX souris 3 rate | 68498 | 32222 |
| Souris 2 rate | | 120064 | 56840 | 40192 | | | pTEX souris 4 rate | 59936 | 28194 |
| Souris 3 rate | | 68498 | 32222 | 22784 | | | Sens souris 1 rate | 2857 | 4041 |
| souris 4 rate | | 59936 | 28194 | 19936 | | | Sens souris 2 rate | 2105 | 2977 |
| | | | | | | | Sens souris 3 rate | 833 | 1179 |

(suite)

Récapitulatif Point 15 j

| | Moyenne Charge parasitaire | Ecart type | Ecart Moyen | | | Moyenne Charge parasitaire | Ecart type Ec |
|---|---|---|---|---|---|---|---|
| Lot 2 Sens | | | | | Sens souris 4 rate | 7059 | 3328 |
| Souris 1 rate | | 2857 | 4041 | 2857 | Sens souris 5 rate | 1333 | 1886 |
| souris 2 rate | | 2105 | 2977 | 2105 | AntiSens souris 1 rate | 12308 | 0 |
| souris 3 rate | | 833 | 1179 | 833 | AntiSens souris 2 rate | 1429 | 2020 |
| souris 4 rate | | 7059 | 3328 | 2353 | AntiSens souris 3 rate | 8000 | 3771 |
| souris 5 rate | | 1333 | 1886 | 1333 | AntiSens souris 4 rate | 6667 | 3143 |
| | | | | | AntiSens souris 5 rate | 8000 | 0 |
| Lot 3 AntiSens | | | | | | | |
| Souris 1 rate | | 12308 | 0 | 0 | | charge parasitaire | ecart type |
| souris 2 rate | | 1429 | 2020 | 1429 | pTEX | 82833 | 3252 |
| souris 3 rate | | 8000 | 3771 | 2667 | Sens | 2838 | 248 |
| souris 4 rate | | 6667 | 3143 | 2222 | AntiSens | 7281 | 390 |
| souris 5 rate | | 8000 | 0 | 0 | | | |

(suite)

| | | | | | | |
|---|---|---|---|---|---|---|
| Parasitémie (2 mois) RATE | | | | | résultats en rouge non pris en compte | |
| | Poids organe (g) | Dilution limite | Charge parasitaire parasite g-1 | Moyenne Charge parasitaire | Moyenne générale Charge parasitaire | Ecart type g |
| Lot 1 pTEX | | | | | | |
| Souris 1 rate | 0,14 | 0,016 | 182857 | | 264127 | 118470 |
| Souris 2 rate | 0,14 | 0,008 | 365714 | | | |
| Souris 3 rate | 0,17 | 0,031 | 75294 | | | |
| souris 4 rate | 0,18 | 0,016 | 142222 | | | |
| souris 5 rate | 0,14 | 0,008 | 365714 | | | |
| Lot 2 Sens | NI= Non infectée | | | | | |
| Souris 1 rate | 0,1 | NI | 0 | | 0 | 0 |
| souris 2 rate | 0,15 | NI | 0 | | | |
| souris 3 rate | 0,12 | NI | 0 | | | |
| souris 4 rate | 0,14 | NI | 0 | | | |
| souris 5 rate | 0,14 | NI | 0 | | | |
| Lot 3 AntiSens | | | | | | |
| Souris 1 rate | 0,11 NI | | 0 | | 1813 | 2914 |
| souris 2 rate | 0,12 | 0,500 | 6667 | | | |

(suite)

| | | | | | | | | | |
|---|---|---|---|---|---|---|---|---|---|
| Lot 3 AntiSens | | | | | | | | | |
| souris 3 rate | | 0,19 | 0,500 | 4211 | | | | | |
| souris 4 rate | | 0,13NI | | 0 | | | | | |
| souris 5 rate | | 0,14NI | | 0 | | | | | |
| souris 6 rate | | 0,18NI | | 0 | | | | | |
| Rate | Charge parasitaire g-1 | | ecart type | | Rate | Charge parasitaire g-1 | | ecart type | |
| | 15 jours | 2 mois | 15 jours | 2 mois | | 15 jours | 2 mois | 15 jours | 2 mois |
| pTEX | 107912 | 264127 | 56756 | 118470 | pTEX | 67348 | 264127 | 22927 | 118470 |
| Sens | 2367 | 0 | 1517 | 0 | Sens | 2367 | 0 | 1517 | 0 |
| AntiSens | 8744 | 1813 | 2458 | 2914 | AntiSens | 8744 | 1813 | 2458 | 2914 |
| Parasitémie (2 mois) | | | | | | | | | |
| FOIE | Poids organe (g) | | Dilution limite | Charge parasitaire parasite g-1 | | | | | |
| Lot 1 pTEX | | | | | | | 2495238 | 4025557avec souris 1 | |
| Souris 1 Foie | | 0,12 | 0,000391 | 8533333 | | | | | |
| Souris 2 Foie | | 0,1 | 0,000024 | 163840000 | | | 482540 | 43989sans souris 1 | |
| Souris 3 Foie | | 0,14 | 0,006250 | 457143 | | | | | |
| souris 4 Foie | | 0,14 | 0,006250 | 457143 | | | | | |
| souris 5 Foie | | 0,12 | 0,006250 | 533333 | | | | | |

(suite)

| | | | | | | |
|---|---|---|---|---|---|---|
| Lot 2 | | | | | | |
| Sens | | | | | | |
| Souris 1 | | 0,17NI | | 0 | 0 | 0 |
| Foie | | | | | | |
| Souris 2 | | 0,18NI | | 0 | | |
| Foie | | | | | | |
| Souris 3 | | 0,12NI | | 0 | | |
| Foie | | | | | | |
| souris 4 | 0,14? | NI | | 0 | | |
| Foie | | | | | | |
| souris 5 | | 0,09NI | | 0 | | |
| Foie | | | | | | |
| | | | | | | |
| Lot 3 | | | | | | |
| AntiSens | | | | | | |
| Souris 1 | | 0,12NI | | 0 | 38889 | 61162 |
| Foie | | | | | | |
| Souris 2 | | 0,16 | 0,025 | 100000 | | |
| Foie | | | | | | |
| Souris 3 | | 0,1 NI | | 0 | | |
| Foie | | | | | | |
| souris 4 | | 0,08NI | | 0 | | |
| Foie | | | | | | |
| souris 5 | | 0,12 | 0,025 | 133333 | | |
| Foie | | | | | | |
| souris 6 | | 0,1NI | | 0 | | |
| Foie | | | | | | |

## Revendications

1. Vecteur d'expression **caractérisé en ce qu'**il comprend un insert, dans une orientation antisens, d'un gène codant pour une PSA de leishmanie d'excrétion/sécrétion.

2. Vecteur selon la revendication 1, **caractérisé en ce qu'**il renferme un insert de gène de *L. amazonensis.*

3. Vecteur selon la revendication 2, **caractérisé en ce que** le gène de *L. amazonensis* répond à l'une des séquences suivantes : SEQ ID N° 1 à 5.

4. Vecteur selon la revendication 1, **caractérisé en ce qu'**il renferme un insert de gène de *L. infantum.*

5. Vecteur selon la revendication 4, **caractérisé en ce que** le gène de *L. infantum* répond à SEQ ID N°6.

**6.** Promastigotes de leishmanies génétiquement modifiés, **caractérisés en ce qu'**il s'agit de mutants avirulents, transfectés par un vecteur selon l'une quelconque des revendications 1 à 5.

**7.** Procédé d'obtention de formes avirulentes de promastigotes, **caractérisée en ce qu'**il comprend :

- l'insertion dans un vecteur d'expression de leishmanies de manière non directionnelle de gènes, codant pour une PSA de leishmanie d'excrétion/sécrétion,
- la sélection des vecteurs renfermant les inserts dans une orientation antisens,
- la transfection de formes des promastigotes de leishmanies à l'aide des vecteurs sélectionnés,
- le clonage biologique par micromanipulation des promastigotes génétiquement modifiés pour l'obtention des mutants sous-exprimant, voire n'exprimant plus, le gène codant pour une PSA de leishmanie.

**8.** Utilisation des promastigotes selon la revendication 7, pour l'élaboration de vaccins.

## Patentansprüche

**1.** Expressionsvektor, **dadurch gekennzeichnet, dass** er ein Insert in Antisense-Orientierung in Form eines Gens umfasst, das für ein *Leishmania*-Exkretions-/Sekretions-PSA codiert.

**2.** Vektor gemäß Anspruch 1, **dadurch gekennzeichnet, dass** er ein Insert in Form eines Gens von *L. amazonensis* umfasst.

**3.** Vektor gemäß Anspruch 2, **dadurch gekennzeichnet, dass** das Gen von *L. amazonensis* einer der folgenden Sequenzen entspricht: SEQ ID Nr. 1 bis 5.

**4.** Vektor gemäß Anspruch 1, **dadurch gekennzeichnet, dass** er ein Insert in Form eines Gens von *L. infantum* umfasst.

**5.** Vektor gemäß Anspruch 4, **dadurch gekennzeichnet, dass** das Gen von *L. infantum* der SEQ ID Nr. 6 entspricht.

**6.** Promastigoten von genetisch modifizierten *Leishmania,* **dadurch gekennzeichnet, dass** es sich um avirulente Mutanten handelt, die mit einem Vektor gemäß einem der Ansprüche 1 bis 5 transfiziert sind.

**7.** Verfahren zur Gewinnung avirulenter Formen von Promastigoten, **dadurch gekennzeichnet, dass** es Folgendes umfasst:

- die ungerichtete Insertion von Genen, die für ein *Leishmania-Exkre*-tions-/Sekretions-PSA codieren, in einen *Leishmania*-Expressionsvektor;
- die Selektion von Vektoren, die Inserts in einer Antisense-Orientierung umfassen;
- die Transfektion von Formen von *Leishmania*-Promastigoten mit Hilfe ausgewählter Vektoren;
- biologische Klonierung durch Mikromanipulation der genetisch modifizierten Promastigoten zur Gewinnung von Mutanten, die das für ein *Leishmania-PSA* codierende Gen unterexprimieren oder sogar nicht mehr exprimieren.

**8.** Verwendung der Promastigoten gemäß Anspruch 7 zur Entwicklung von Impfstoffen.

## Claims

**1.** An expression vector, **characterized in that** it comprises an insert, in an antisense orientation of a gene coding for an excreted/secreted PSA of *Leishmania.*

**2.** The vector according to claim 1, **characterized in that** it contains an *L. amazonensis* gene insert.

**3.** The vector according to claim 2, **characterized in that** the *L. amazonensis* gene corresponds to one of the following sequences: SEQ ID Nos. 1 to 5.

**4.** The vector according to claim 1, **characterized in that** it contains an *L. infantum* gene insert.

**5.** The vector according toclaim 4, **characterized in that** the *L. infantum* gene corresponds to SEQ ID No. 6.

**6.** Genetically modified leishmania promastigotes, **characterized in that** they are avirulent mutants, transfected with a vector according to any one of claims 1 to 5.

**7.** A method of obtaining avirulent forms of promastigotes, **characterized in that** it comprises:

- inserting, into a *Leishmania* expression vector, in a nondirectional orientation, genes coding for an excreted/secreted PSA of *Leishmania,*
- selecting the vectors containing the inserts in an antisense orientation,
- transfection of *Leishmania* promastigote forms using the selected vectors,
- biological cloning of the genetically modified promastigotes by micromanipulation to obtain mutants which underexpress, or even which no longer express, the gene encoding a *Leishmania* PSA.

**8.** The use of the promastigotes according to claim 7, for developing vaccines.

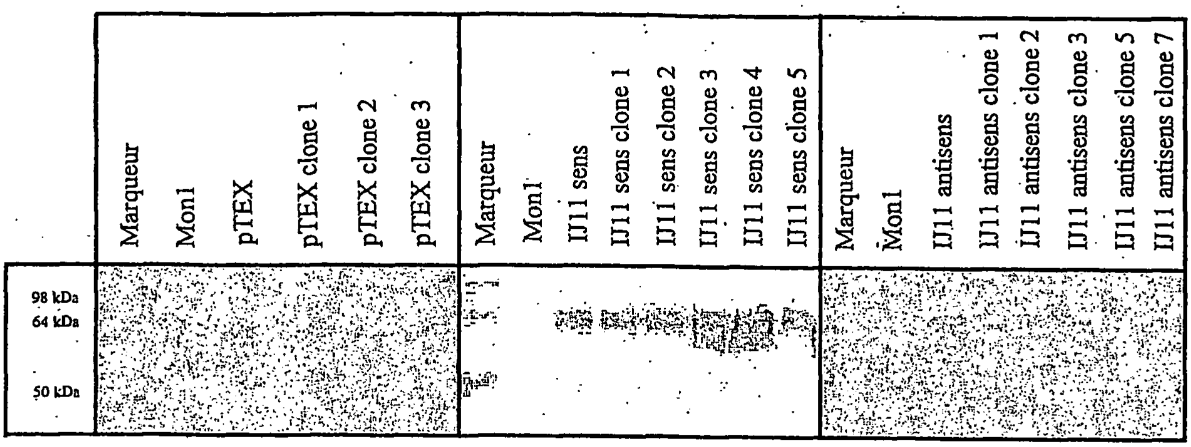


Figure 1

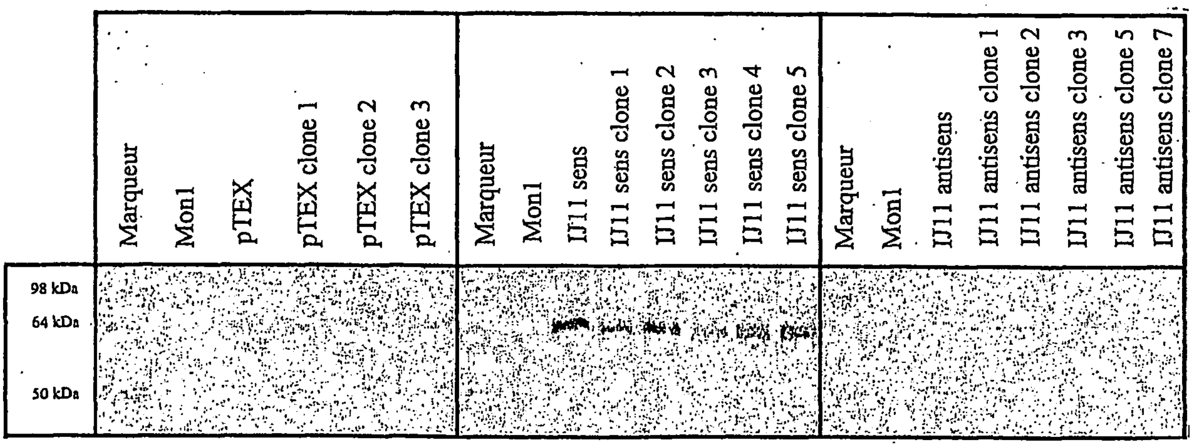


Figure 2

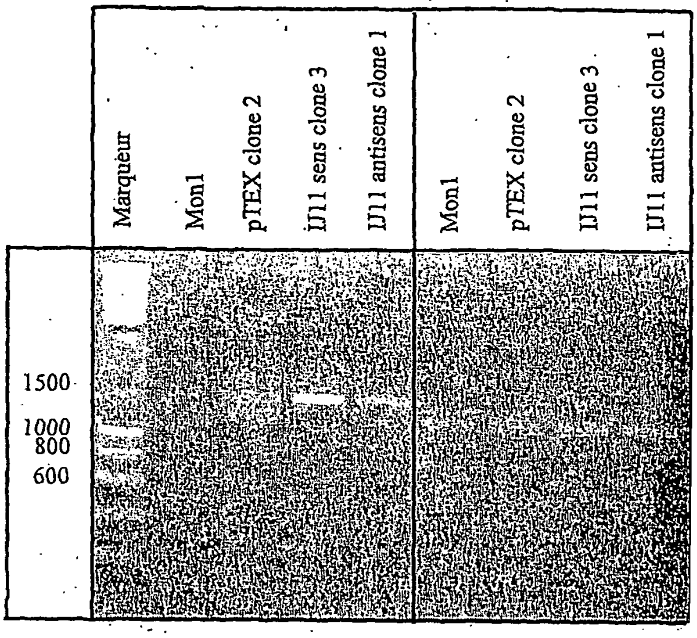


Figure 3

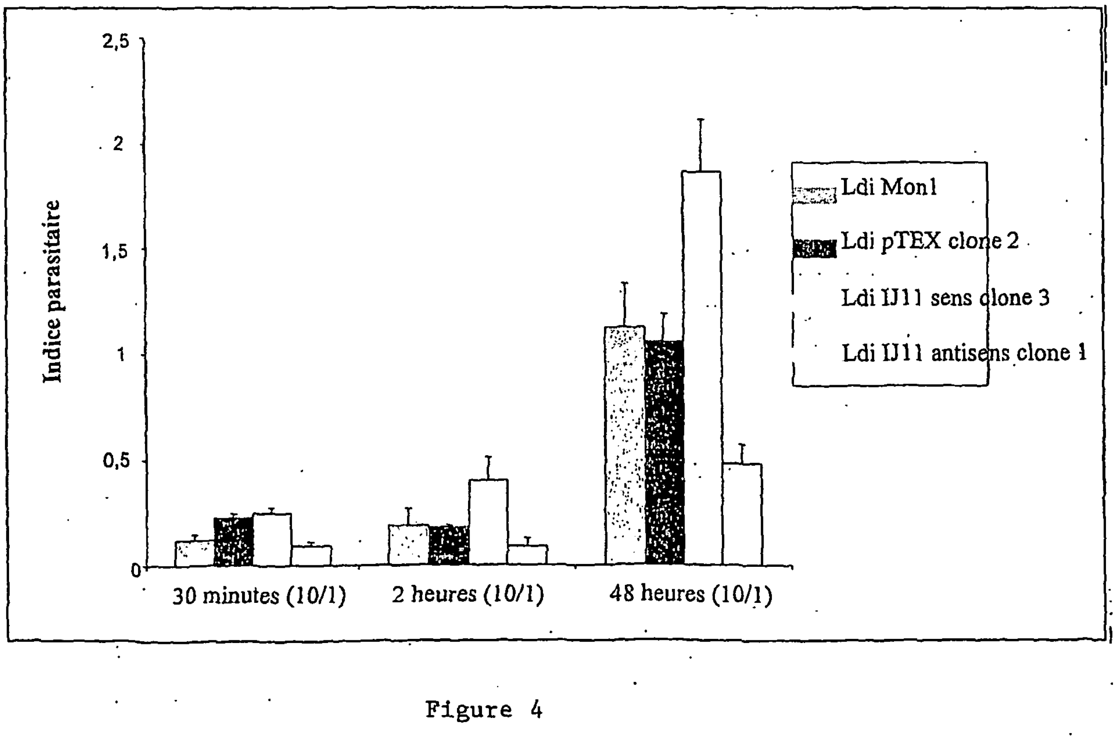


Figure 4

SEQ ID N°1 (Séquence nucléique A3B)

GACCCCTGTTGCGAATGGCGCAGTGCGTGCGTCGGCTGGTGCTGGGGGCGCCCTCGCGGCTGTGG
TGGCGCTGCTGCTGTGCACGAGCAGTGCACCGGTGGGCGTGCTGCGGGGACGAGCCACTTCACTG
AGGCGCAGCAGACGAACACGGTGACGGTGCTGCAGGCGTTTGCGCGTGCGATCCGTGCGCTTGGG
GACACCGTGGACGGGCAGCGACTTCTGCTCGTGGAAGCACATCATCTGCGACTGCCCCGGCGTCGGC
GTGTGGATGGGCGATGTGGATTATACCGGCACGGTGCGGGAGATGCCTGCGAGCGTCGACTACAA
GGACGTCATGATCACGGAACTGAACTTCAGCGCAATGGGCCAGGGGCTGAGCGGGACGCTGCCCG
CCTCATGGAGCTCGCTGACGTCCTTGATATGACTGTGCATCGAAAAGTCTGAGAAGGTCACCGGCA
CGCTGCCTGCCCAGTGGAGCTCGATGACGTCGCTGGACAACCTTAACCTGCACGACACGGCGGTCT
CGGCCACGCTGCCTGCCCAGTGGAGCTCGATGAAGCAGCTGACCGTTCTGGATCTGGAGGGCACTA
AGGTGTCCGGCACGCTGCCGTCCGAGTGGAGTGGGATGGCGAAGGCCGAGGCCGTGCAGCTGGAG
AACTGCGGTCTGTCCGGGAGTCTGCCCCCCTCGTGGTCTGCGATGCCGAAGCTGCGTATCGTCTCAC
TGAGCGGCAACCACTTCTGCGGCTGCGTGCCCGACTCGTGGAGGGAGAAGGACCGCCTCGATGTC
ACCATCGAGGAATGGCACATGGGCGAGGACTGCAAGCTTGCTAACGCCTGCCGCCCGACTGCTGCT
CCGGGAACGACCACGACTAACCCGCCCACCACCACCGGCACCGCAGCAGCCTCCTCTACTCCTTCT
CCAGGGTCGGGGTGCGAGGTGGATGGCTGTGAGGTGTGCGAGGGGGACTCCGCTGCGCGGTGCGC
CAGGTGCCGTGAGGGCTACTCCCTGACGGACGAGAAGACGTGCCTGGCGAACCACCATGGCGGCG
TGGCGGCGGCGTCGAGCGGAGCGGTGGCTGCCGCTGCTGTGTGGGCGGCTGTGCTGTTGAGCGTGG
GGCTGGTGGCGTGAGGGTGCGGCGGGGCCCGTCTTCTCTGTGGTGCCCCTGCTGCCTGCCCTCGCCC
CCGGCACGGCGTCGTCGCTGCCCTCTCTCACCCCCACCAGCCGACGGGGAGACCGACAGCCACACG
CGCACGCGCACACGCCGTCGTGCATCGCGTGTGCTTTCGGCCGTTGTGGCGCCTGCAGGGATGCAC
GGGCATGCGGAGGCGTGCATGCGTGTGGGGGTGCCAGCTCTTGTGTGTCTCTCCGTGTGGCCAGCA
GTCGGCACGCGCCGCCGATCGAATGTGCGCCGCGGCGGCGGTGTGTCGGCCTTGGACAGCGGATGCGG
GCGGCCCGCCGCCTCGGCGTGTGCCCTGCGGTGTGCTGTGCTGCCGGCGCGAGGGACGTACGGATGCGC
TGTCCGGCCCTCTTCGACGGGGCTCGCTTGCGGTGCTGTGCTCTCGTGGTCTGTGCCGGTGCTGCCC
TGGCGGGGTGAGAGCTGGCGGGGGCGTGGGTGCGCGCGCGGCAGCTCTCCGCTGCGTTGAGGGCG
GCCTGCCCCTGCGTCCGCGCACCGTCGCGCTCTCCTCGACGCCACTGCGCGCGCTTGTTGGCTTGCT
TTGCTCTGTCGTGCGCACTCTCTCTTATTTTCCGTTTCATTCGCCTGTATTCTCTTCTCCCACCGCACT
GCGGCCTCGTCACCGCGGCCGTGCGGTGCGCAGGCGGGTGATGTGCCGTTGTGCCCCCCCTTTCAT
GGCGCGCTGGGCCGATCGCCCTCTTGCCTCCCTCCTCCCCCTCCCCCTCCCGCCGGTCCTGTCAATT
GTATATCCGTGGACCTTATCTTCGTACTGCCTCCGCGCCTCTTCCGTAAAGCTTCGTTGGCGTGTGC
CGCCCCCCCGGACGTCAGCGCCCGCTGTGCTCGCATGCTCACGGTGCGTCCCCGTGCGTGGGCGTGCA
CGTAAGGACATGTATATATGTATGTGTATGTATATGAGTATGTATATATGTACGGTTATATATAGGA
ATTTGTGTATGTTGAGGTGTATGCATGTGCGTGCGTATATTAGTGTGTGCGAGCACGCGTGTTGCGC
CACGCTCTGCTGCCCGCCTCCGCTGTGCGTGTCACTCGCTGTGGGCGCGGTGGCGGGTGGCGCCGG
GTGGTGGCCGTGCGGCGGGCGGGGGCTCCTCTGTGTTTCTCTATTTCTCTGTTCCCTGTTGACCTCA
AAAAAAAAAAAAAAAAAAAA

SEQ N° 2 (séquence nucléique 2C1)

CGTGGACGGGCAGCGACTTCTGCTCGTGGAAGCACATCATCTGCGACTCCCCGGCGTCGGCGTGT
GGATGGGCGATGTGGATTATACCGGCACGGTGCGGGAGATGCCTGCGAGCGTCGACTACAAGGAC
GTCATGATCACGGAACTGAACTTCAGCGCAATGGGCCAGGGGCTGAGCGGGACGCTGCCCCCCTC
ATGGAGCTCGCTGACGTCCTTGATATCACTGTGCATCGAAAAGTCTGAGAAGGTCACCGGCACGCT
GCCTGCCCAGTGGAGCTCGATGACGTCGCTGGACAACCTTAACCTGCACGACACGGCGGTCTCCGG
CACGCTGCCTGCCCAGTGGAGCTCGATGAAGCAGCTGACCGTTCTGGATCTGGAGGGCACTAAGGT
GTCCGGCACGCTGCCGTCCGAGTGGAGTGGGATGGCGAAGGCCGAGGCCGTGCAGCTGGAGAACT
GCGGTCTGTCCGGGAGTCTGCCCCCCTCGTGGTCTGCGATGCCGAAGCTGCGTATCGTCTCACTGA
GCGGCAACCACTTCTGCGGGTGCGTGCCCGACTCGTGGAGGGAGAAGGACCGCCTCGATGTGACC
ATCGAGGAATGGCACATGGGCGAGGACTGCAAGCTTGCTAACGCCTGCCGCCCGACTGCTGCTCCG
GGAACGACCACGACTAACCCGCCCACCACCACCGGCACCGCAGCAGCCTCCTCTACTCCTTCTCCA
GGGTCGGGGTGCGAGGTGGATGGCTGTGAGGTGTGCGAGGGGGACTCCGCTGCGCGGTGCGCCAG
GTGCCGTGAGGGCTACTCCCTGACGGACGAGAAGACGTGCCTGGCGAACCACGATGGCGGCGTGG
CGGCGGCGTCGAGCGGAGCGGTGGCTGCCGCTGCTGTGTGGGCGGCTGTGCTGTTGAGCGTGGGGC
TGGTGGCGTGAGGGTGCGGCGGGGCCCGTCTTCTCTGTGGTGCCCCTGGTGCCTGCCCTCGCCCCCG
GCACGGCGTCGTCGCTGCCCTCTCTCACCCCCACCAGCCGACGGGGAGACCGACAGCCACACGCGC
ACGCGCACACGCCGTCGTGCATCGCGTGTGC

SEQ N° 3 (séquence nucléique 1A1)

GGACGGGCAGCCGAGTTCTGGTCGTGGAAGCACATCATCTGCGACTCCCCGGCGTCGGCGTGTGGA
TGGGCGATGTGGATTATACCGGCACGCTGCCGGAGATGCCTGCGAGCGTCGACTACAAGGACGTG
ATGATCATGGCACTGGACTTCGGCGGAATGGGCCAGGGACTGAGCGGGACGCTGCCCCCCTCATGG
AGCTCGCTGACGTCCTTGATGTCACTGTGGATCGAAAAGTCTGACAAGGTCACCGGCACGCTGCCT
ACCCAGTGGAGCTCGATGAAGCAGCTGACCCTTCTGCATCTGAAGGGCACTAAGGTGTCCGGCACG
GTGCCGCCCGAGTGGAGTGGATGACGTGGCTGGACGACCTTAACCTGCACGACACGGCGGTCTCG
GGCACGCTGCCTGCGCAGTGGAGCTCGATGAAGCAGCTGATCGATCTGGATCTGGAGGGCACTAA
GGTGTCCGGCACGCTGCCGCCCGAGTGGAGTGGGATGGCGAAGGCCGAGGCCCTGCAGCTGAAGT
ACTGCGATCTGTCCGGGAGTCTGCCCCGCTCGTGGTCTTCGATGGAGAAGCTGCGTATCGTCTCACT
GAGCGGCAACCACTTCTGCGGGTGCGTGCCCGACTCGTGGAGGGAGAAGGACCGCCTCGATGTGA
CCATCGAGGAATGGCACATGGGCGAGGACTGCAAGCTTGCTAACGCCTGCCGCCCGACTGCTGCTC
CGGGAACGACCACGACTAACCCGCCCGACCACCACCGGCACCGCCAGCAGCCTCCTCTACTCCTTCTG
CAGGGTCGGGGTGCGAGGTGGATGGGTGTGAGGTGTGCGAGGGGGACTCCGCTGCGCGGTGCGCG
AGGTGCCGTGAGGGCTACTCCCTGACGGACGAGAAGACCGTGCCTGGCGAACCACGATGGCGGCGT
GGCGGCGGCGTCGAGCGGAGCGGTGGCTGCGGCTGCTGTGTGGGCGGCTGTGCTGTTGAGCGTGG
GGCTGGTGGCGTGAGGGTGCGGCGGCCCCCTCTTCTCTGTGGTGCCCCTGGTGCCTGCCCTCGCCG
CCAGCACGGCGTCGTCGCTGCCCTCTCACCCCCAGCAGCGGAAGGGGAGACCGACAGCCACAGGC
ACACGCGGACGCGCCGTCGTGCATCGCGTGTGCTTTCCGCCGTTGTGGCGCCTGCGCGGATGCACG
GGCATGCGGAGGCGTGCATGCGTGTGCCGGTGCCAGCTCTTGTGTGTCTCTCCGTGTGGCCAGCAG
TCGGCAGCCGGCGCCGATCGAATGTGGCGGCGGCGGCGGTGTGTCCGCCTTGGACAGCGGATGCCGG
GCCCGCCCCTCGCCGTGTGCCCTGCGGTCTGCTGTGCTGGCGGCGCAGCGACGTACGGA

SEQ N°4 (Séquence nucléique 2G1)

TCGGCGTGTGGATGGGCGATGTGGATTATACCGGCACGCTGGCGGAGATGCCTGCGAGCGTCGACT
ACAAGGACGTCATGATCACGGAACTGAACTTCGGCGGCAATGGGCCAGGGACTGAGCGGGACGCTG
CCCCCCTCATGGAGCTCGATGAAGCAGCTGATCGATCTGGATCTGGAGGGCACTAAGGTGTCCGGC
ACGCTGCCGCCCGAGTGGAGTGGGATGGCGAAGGCCGAGGCCCTGCAGCTGAAGTACTGCGATCT
GTCCGGGAGTCTGCCCCCGCTCGTGGTCTTCGATGGAGAAGCTGCGTATCGTCTCACTGAGCGGCAA
CCACTTCTGCGGGTGCGTGCCCGACTCGTGGAGGGAGAAGGACCGCCTCGATGTGACCATCGAGG
AATGGCACATGGGCGAGGACTGCAAGCTTGCTAACGCCTGCCGCCCGACTGCTGCTCCGGGAACG
ACCACGACTAACCCGCCCGACCACCACCGGCACCCCAGCAGCCTCCTCTACTCCTTCTCCAGGGTCG
GGGTGCGAGGTGGATGGGTGTGAGGTGTGCGAGGGGGACTCCGCTGCGCGGTGCGCCAGGTGCCG
TGAGGGCTACTCCCTGACGGACGAGAAGACGTGCCTGGCGAACCACGATGGCGGCGTGGCGGCGG
CGTCAAGCCGCAGCCGGTGGCTCCGGCTGCTCTGTGGGCGGCTGTGCTGTTGAGCGTGGGGCTGGTGG
CGTGAGGGTGCGGCGGGCCCCTCTTCTCTGTGGTGCCCCTGGTGCCTGCCCTCGCCCCCGGCACGG
CGTCGTCGCTGCCCTCTCTCACCCCCACCAGCCGACGGGGAGACCGACAGC
CACACGCGGCACGCGCACACGCCCGTCGTGCATCGCGTGTGCTTTCCGCCGTTGTGGCGCCTGCACGG
ATGCACGGGCATGCGGAGGCGTGCATGCGTGTGCGCGTGCCAGCTCTTGTGTGTCTCTCCGTGTGG
CCAGCAGTCGGCAGCCGGCGCCGATCGAATGTGCGGCGGCGGCGGCGGTGTGTCCGCCTTGGACAGCGG
ATGCTGGCGGCCGCGCCCCTCGCCGTGTGCCCTCGGTCTGCCGTGTCGTGGCCGGCGCGAGCGACGTACGGA
GTGCGGTGTCGCCGGGTGGTGGCCGTGCGGCGGGCGGGGGCTCCTCTGTGTTTCTCTATTTCTCTGT
TCCCTGTTGACCTCAAAAAAAAAAAAAAAAAAAAAA

SEQ N° 5 (Séquence nucléique W2)

CGGGCGTCGGCGTGTGGATGGGCGATGTGGATTATACCGGCACGCTGCCGGAGAT
GCCTGCGGAGCGTCGACTACAAGGACGTCATGATCACGGAACTGAACTTCAGCGC
AATGGGCCAGGGGCTGAGCGGGACGCTGCCCCCCTCATGGAGCTCGGTGACGTCC
TTGATATCACTGTGCATCGAAAAGTCTGAGAAGGTCACCGGCACGCTGCCTGCCC
AGTGGAGCTCGATGACGTCGCTGGACAACCTTAACCTGCACGACACGGCGGTCTC
CGGCACGCTGCCGCCCGGAGTGGAGTGGGATGAGGTCGCTGGACGACCTTAACCTG
CACGACACGGCGGTCTCCGGCACGCTGCCTGCCCAGTGGAGCTCGATGAAGCAG
CTGATCGATCTGGATCTGGAGGGCACTAAGGTGTCCGGCACGCTGCCGCCCGAGT
GGAGTGGGATGGCGAAGGCCGAGGCCCTGCAGCTGAAGTACTGCGATCTGTCCG
GGAGTCTGCCCCCCTCGTGGTCTTGGATGGAGAAGCTGGCGTATCGTCTCACTGAG
CGGCAACCACTTCTGCGGGTGCGTGCCCCGACTCGTGGAGGGAGAAGGACCGCCT
CGATGTGACCATCGAGGAATGGCACATGGGCGAGGACTGCAAGCTTGCTAACGC
CTGCCGCCCGACTGCTGCTCCGGGAACGACCACGACTAACCCGCCCAGCACCAGC
GGCACCCCAGCAGCCTCCTCTACTCCTTCTCCAGGGTCGGGGTGCGAGGTGG
ATGGGTGTGAGGTGTGCCGAGGGGGACTCGGCTGCGCGGTGCGCCAGGTGCCGTG
AGGGCTAGTCCTGACGGACGAGAAGACGTGCCTGGCGAACCACGATGGCGGCGT
GGCGGCGGCGTCAAGCGGAGCGGTGGCTGCGGCTGCTGTGTGGGCGGCTGTGCT
GTTGAGCGTGGGGCTGGTGGCGTGAGGGTGCCGCCGCCCCCTCTTCTCTGTGGTG
CCCCTGGTGCCTGCCCTCGCCCCCAGCACGGGGTCCTCGCTGCCCTCTCACCCCA
CCAGCCGAAGGGGAGACCGACAGGCACACGCAGACGCGGCACGCGCCGTCGTGCA
TCGCCGTGTGCTTTCCGCCGTTGTGGCGCCTGCGGGATGCACGGGCATGCGGAGG
CGTGCATGCGTGTGCGCGTGGCAACTCTTGTGTGTCTCTCCGTGTGGCCAGCAGTC
GGCACCC

SEQ N°6 (Séquence nucléique IJ11 de Ldi)

GCGCTGCTGCCGCTGGCGCTGTTGTGTGTGTGCTGGGGCCGCGCCACGCA
CACGCACGGTAGTGAGGGGGAGCCGCAGCGACCGACCGGGCGGAGCGGGC
GGGCGGAGGGGGGCGCTCCCGCCCGCTGGTCATGCTCTCTGTTTCGCTGG
CCGGCCTCTCTACGCCGCTGGCGTGGGCGGAGCTCCGCGCTGCGTATCGC
TCGCCCCTCGCTGCCCCTCCCTGCCCCTCCTCATGTGCACTGCTCCCTCC
CTCTCCCTCCCTCTACACTCCTCGCTGTCCCCTCGGCCGACCTCCACGGA
CACGCAGACGTGCGTGCGCATACACACCACCCCTCACCTCGCTGCTGCTG
CTGTGACAGCTCTACGGACCCTGCCCAGTCGCTGCGCCCCCGCCACCCGC
CTCTGTCCCCCGCACGAGGGTACCTACGACGTGCCGGCCACCCCGCTCTG
CCCGATAAGCTGAGCTGGCGCTCACGCCCGAGCAATCCCCTCACGGATCT
GCTGCCGCGCCGCACTGCTCTTGACCCTGGCTGCGAATGGCGCTGTGCGT
GCGTCGGCTGGTGCTGGCGGCGACCCTCGCCGCTGTGGTGGCGCTGCTGC
TGTGCACGAGCAGTGCGCCGGTGGCGCGTGCTGCTGTGAAGGATGACTTC
ACTGCTGCGCAGCGGACGAACACGCTGGCGGTGCTGGAGGCGTTTGGGCG
TGCGATCCCTGAGCTTGGGAAGCTGTGGAAGGGCGACGACTTCTGC
TTTTGGGAGTCGGTCGTGTGCGATGTGACCGAAGTGTACTTGTGGGAA
ATCGGTGCGACGTATACCGGCACGCTGCCGGAGATGCCTGTGGACGTCGA
CTACACGGCCGTCATGGTCAAGCACCTCGACTTTTCCCAAATGGGGCTGG
GGCTGAGCGGAACGCTGCCGGACAGCTGGAGCAGGCTGCAGGGACTGACC
TCACTTACGTTGTCGGGCTGCGGCGTGAGCGGTACGCTGCCCCCCTCGTG
GCGCTCGATGAAGTCTTTGGTGTCGTTGTGGATTGAGAGTTGTGAAAGTG
TTACCGGCAAGCTGCCGCCTGAGTGGAGCTCGATGAAATCGCTGAGAGAT
CTCCATCTGCATGGCGCGAAGGTTTCCGGCACGCTGCCGCCTGAGTGGAG
CACGATGAAATCGCTGACCCTTCTCGATCTGCAGGACACTCAGGTTACCG
GCAGTCTGCCGCCTGAGTGGAGCTCAATGAAATCCATGACCATTCTCAGT
CTGAATGGCGCGAAGGTTTCCGGCACGCTGCCACCCCAGTGGAGCTCGAT
GACATCGCTGAGCCTTCTCAGTCTGGAGGGTACTCAGCTCTCCGGCACGC
TACCGCCCCAGTGGAGTGGGATGACATCGCTGGTCACGCTTTTTCTGCA
GGGTACTCAGGTCTCCGGCACTCTGCCGCCGCAGTGGAGATCGATGTTGAA
TGCCGAGTTCCTGCAGCTGGAGAACTGCGACCTGTCCGGCTGTTTGCCCCC
CGAGTGGGCTGCGATGCCGAAGCTGCGTCATGTCGAACTTAAGGGCAACCA
GTTCGCCGGGTGTGTGCCGGACTCGTGGGCTCAGAAGGCCGGTCTCGTTGT
GGAAATCGAGGATAAGCACACGGGCAACAGCTGCATTGCTGGTGCGGACTG
CGCAACGACGACCACGACCACCACTGAACCCACGTCCACTGCGAGCCCAAC
AGCCACGCCTACCTCTGCCCCCGAGACGGAGTGCGAGGTGGATGGGTGTGA
GGTGTGCGATGGGGACTCCGCGGCGAGGTGCGCCAGGTGCCGTGAGGGCTA
CTTCCTGACGGACGAGAGGACGTGCCTGGTGTACCGCGATGGCGGCGTTGT
GGCCGTGTCGATCGGAGCGGCTGCTGCCGCTGTTGTGTGCATGGCTGTGCT
GCTGAGCGTGGGGCTGGCGGCGTGAGGATGCCGCTGCTGTCGCGCGCAGGC
GGCGGCACCCGCTGCGTGGCACACGACTGCGTGCTTGCGTGCAGCACCGCG
CCCTGCATTGGCGTGCGTGTGCGCGTCTGTGTGTGCATGGCTGCTGACGGT
GCCTTTCGTCCTGCCTCTCGCTGCCTCTGCCTCTCTCCGCGTGTGAATGCT
GTGGGCTGTGTTTGGGGCTCTCGTGCGGCGCTGCTGTACGGCTGCTGCTTC
TTCTCCACCCTCCTCTCTCGCATGCCGGCGAGGGAGGGGTGGCACGTGCGC
GTGTGCCGCTGCGCTTGCGAGTGCGTCTGTGTGTGGGCCTTCACCACGTGC

TACGGTCACGCCTTCTCGGCTGGCCACTCGCGGCGCTGAGGGCGGTGTGCC
CTTCCCCTCGAGCGCCGTCGCACTCTCTTCCGCGCGCCTGCGCGGGCTTCT
TCGTGCGCTGTGCTCAGCCGTGCGCTCTCACCTCTTTCCCTTTTCATTCGC
TTGTCTTCTCTCTTCTCCCCCCGCACTGCGGTCTCCCCTCCTCTGCCGTGC
GGTGCGCAGGCGGGTGACTTGCCGTTGCGTCTCCCCCTTTCGTGGAGCGCT
GAGCCGATCCCCCTTCGGCCTCCCTCCTCCCTCCTCCCGTGGGTCCTGTCT
GTTGTACATCGTCGGACCGTCTCTTCGTGTTGCCTCTCCGCACCTTCCGCA
AATCTGCGCTCGCCTGTGCCGCCTCTCGGACTTTATCCTTACTGTGATTGT
ATTCTCACGGTGCGTCTCCGTGTGTGTGTGTGCCACGCACCGCTTCTTCCA
TGTGTGTCCTTGCTTGCTCTCGTCTGCCCCCCCCCCTCTGCCTCACACATT
CCGTGCGTGTGTGCATCACCGTTGGGCGGCGACATCGGTGCCCGTCCCTGC
CACCCTCTACTCCCTCATTCTCTTGCCACTTCGTGGGCGGTGCGTGCATGC
ATGGATGTATATACACGCATAGAGGGGTGGGGACGCGGGGGATCCTCTAGA
GTCGACCTGCAGGCATGCAAGCTTGGCGTAATCATGGTCATAGCTGTTTCC
TGTGTGAAATTGTTATCCGCTCACAATTCCACACAACATACGAGCCGGAAG
CATAAAGTGTAAAGCCTGGGGTGCCTAATGAGTGAGCTAACTCACATTAAT
TGCGTTGCGCTC

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 9305779
- FR 0501348


### Littérature non-brevet citée dans la description


- **CHEN et al.** *Infection and Immunity,* vol. 200, 80-86
- **HAJMOVÀ et al.** *Microbes and Infection,* vol. 6 (20.04), 646-649
- **LOHMAN et al.** *P.N.A.S. USA,* 1990, vol. 87, 8399-8397
- **BUFFET P.A. et al.** *Antimicrobial agents and chemotherapy,* 1995, vol. 39, 2167-2168

SEQUENCE LISTING

<110> INSTITUT DE RECHERCHE POUR LE DEVELOPPEMENT (IRD)

<120> MOYENS POUR L'OBTENTION DE PROMASTIGOTES DE LEISHMANIES VIRULENTS, PROMASTIGOTES OBTENUS ET LEURS APPLICATIONS

<130> CP/BB 61497-2123

<140> FR 05 01 348
<141> 2005-02-10

<160> 10

<170> PatentIn version 3.1

<210> 1
<211> 2333
<212> DNA
<213> Artificial séquence

<220>
<223> Leishmania amazonensis promastigotes surface antigens

<400> 1

```
gacccctgtt gcgaatggcg cagtgcgtgc gtcggctggt gctggcggcg cccctcgccg      60
ctgtggtggc gctgctgctg tgcacgagca gtgcaccggt ggcgcgtgct gcggggacga     120
gcgacttcac tgaggcgcag cagacgaaca cgctgacggt gctgcaggcg tttgcgcgtg     180
cgatccctgc gcttggggac acgtggacgg gcagcgactt ctgctcgtgg aagcacatca     240
tctgcgactc cccggcgtc ggcgtgtgga tgggcgatgt ggattatacc ggcacgctgc     300
cggagatgcc tgcgagcgtc gactacaagg acgtcatgat cacggaactg aacttcagcg     360
caatgggcca ggggctgagc gggacgctgc ccccctcatg gagctcgctg acgtccttga     420
tatcactgtg catcgaaaag tctgagaagg tcaccggcac gctgcctgcc cagtggagct     480
cgatgacgtc gctggacaac cttaacctgc acgacacggc ggtctccggc acgctgcctg     540
cccagtggag ctcgatgaag cagctgaccg ttctggatct ggagggcact aaggtgtccg     600
gcacgctgcc gtccgagtgg agtgggatgg cgaaggccga ggccgtgcag ctggagaact     660
```

gcggtctgtc cgggagtctg cccccctcgt ggtctgcgat gccgaagctg cgtatcgtct 720
cactgagcgg caaccacttc tgcgggtgcg tgcccgactc gtggagggag aaggaccgcc 780
tcgatgtgac catcgaggaa tggcacatgg gcgaggactg caagcttgct aacgcctgcc 840
gcccgactgc tgctccggga acgaccacga ctaacccgcc caccaccacc ggcaccccag 900
cagcctcctc tactccttct ccagggtcgg ggtgcgaggt ggatgggtgt gaggtgtgcg 960
agggggactc cgctgcgcgg tgcgccaggt gccgtgaggg ctactccctg acggacgaga 1020
agacgtgcct ggcgaaccac gatggcggcg tggcggcggc gtcgagcgga gcggtggctg 1080
ccgctgctgt gtgggcggct gtgctgttga gcgtggggct ggtggcgtga gggtgcggcg 1140
ggcccctctt ctctgtggtg cccctggtgc ctgccctcgc ccccggcacg gcgtcgtcgc 1200
tgccctctct cacccccacc agccgacggg gagaccgaca gccacacgcg cacgcgcaca 1260
cgccgtcgtg catcgcgtgt gctttccgcc gttgtggcgc ctgcacggat gcacgggcat 1320
gcggaggcgt gcatgcgtgt gcgcgtgcca gctcttgtgt gtctctccgt gtggccagca 1380
gtcggcaccc gcgccgatcg aatgtgcgcg cggcggcggt gtgtcgcctt ggacagcgga 1440
tgcgggcgcc cgcccctcgc cgtgtgccct gcggtctgct gtgctgccgc gcgagcgacg 1500
tacggatgcg ctgtccggcc ctcttcgacg gggctcgctt gcggtgctgt gctctcgtgg 1560
tctgtgccgg tgctgccctg gcggggtgag agctggcggg ggcgtgggtg cgcgcgcggc 1620
agctctccgc tgcgttgagg gcggcctgcc cctgcgtccg cgcaccgtcg cgctctcctc 1680
gacgccactg cgcgcgcttg ttggcttgct ttgctctgtc gtgcgcactc tctcttattt 1740
tccgtttcat tcgcctgtat tctcttctcc caccgcactg cggcctcgtc accgcggccg 1800
tgcggtgcgc aggcgggtga tgtgccgttg tgccccccct ttcatggcgc gctgggccga 1860
tcgccctctt gcctccctcc tccccctccc cctcccgccg gtcctgtcaa ttgtatatcc 1920
gtggacctta tcttcgtact gcctccgcgc ctcttccgta aagcttcgtt ggcgtgtgcc 1980
gccccccgga cgtcagcgcc gctgtgctcg catgctcacg gtgcgtcccc gtgcgtgggc 2040
gtgcacgtaa ggacatgtat atatgtatgt gtatgtatat gagtatgtat atatgtacgg 2100
ttatatatag gaatttgtgt atgttgaggt gtatgcatgt gcgtgcgtat attagtgtgt 2160
gcgagcacgc gtgttgcgcc acgctctgct gcccgcctcc gctgtgcgtg tcactcgctg 2220
tgggcgcggt ggcgggtggc gccgggtggt ggccgtgcgg cgggcggggg ctcctctgtg 2280
tttctctatt tctctgttcc ctgttgacct caaaaaaaaa aaaaaaaaaa aaa 2333

<210> 2
<211> 1081
<212> DNA
<213> Artificial sequence

<220>
<223> Leishmania amazonensis promastigotes surface antigens

<400> 2

```
cgtggacggg cagcgacttc tgctcgtgga agcacatcat ctgcgactcc cccggcgtcg     60
gcgtgtggat gggcgatgtg gattataccg gcacgctgcc ggagatgcct gcgagcgtcg    120
actacaagga cgtcatgatc acggaactga acttcagcgc aatgggccag ggctgagcg     180
ggacgctgcc cccctcatgg agctcgctga cgtccttgat atcactgtgc atcgaaaagt    240
ctgagaaggt caccggcacg ctgcctgccc agtggagctc gatgacgtcg ctggacaacc    300
ttaacctgca cgacacggcg gtctccggca cgctgcctgc ccagtggagc tcgatgaagc    360
agctgaccgt tctggatctg gagggcacta aggtgtccgg cacgctgccg tccgagtgga    420
gtgggatggc gaaggccgag gccgtgcagc tggagaactg cggtctgtcc gggagtctgc    480
ccccctcgtg gtctgcgatg ccgaagctgc gtatcgtctc actgagcggc aaccacttct    540
gcgggtgcgt gcccgactcg tggagggaga aggaccgcct cgatgtgacc atcgaggaat    600
ggcacatggg cgaggactgc aagcttgcta acgcctgccg cccgactgct gctccgggaa    660
cgaccacgac taacccgccc accaccaccg gcaccccagc agcctcctct actccttctc    720
cagggtcggg gtgcgaggtg gatgggtgtg aggtgtgcga gggggactcc gctgcgcggt    780
gcgccaggtg ccgtgagggc tactccctga cggacgagaa gacgtgcgtg gcgaaccacg    840
atggcggcgt ggcggcggcg tcgagcggag cggtggctgc cgctgctgtg tgggcggctg    900
tgctgttgag cgtggggctg gtggcgtgag ggtgcggcgg gcccctcttc tctgtggtgc    960
ccctggtgcc tgccctcgcc cccggcacgg cgtcgtcgct gccctctctc acccccacca   1020
gccgacgggg agaccgacag ccacacgcgc acgcgcacac gccgtcgtgc atcgcgtgtg   1080
c                                                                   1081
```

<210> 3
<211> 1371
<212> DNA
<213> Artificial sequence

<220>
<223> Leishmania amazonensis

<400> 3

```
ggacgggcag cgacttctgc tcgtggaagc acatcatctg cgactccccc ggcgtcggcg     60
tgtggatggg cgatgtggat tataccggca cgctgccgga gatgcctgcg agcgtcgact    120
acaaggacgt catgatcatg gcactggact tcggcgcaat gggccaggga ctgagcggga    180
cgctgccccc ctcatggagc tcgctgacgt ccttgatgtc actgtggatc gaaaagtctg    240
agaaggtcac cggcacgctg cctacccagt ggagctcgat gaagcagctg acccttctgc    300
```

```
atctgaaggg cactaaggtg tccggcacgc tgccgcccga gtggagtggg atgacgtcgc   360
tggacgacct taacctgcac gacacggcgg tctccggcac gctgcctgcc cagtggagct   420
cgatgaagca gctgatcgat ctggatctgg agggcactaa ggtgtccggc acgctgccgc   480
ccgagtggag tgggatggcg aaggccgagg ccctgcagct gaagtactgc gatctgtccg   540
ggagtctgcc cccctcgtgg tcttcgatgc agaagctgcg tatcgtctca ctgagcggca   600
accacttctg cgggtgcgtg cccgactcgt ggagggagaa ggaccgcctc gatgtgacca   660
tcgaggaatg gcacatgggc gaggactgca agcttgctaa cgcctgccgc ccgactgctg   720
ctccgggaac gaccacgact aacccgccca ccaccaccgg caccccagca gcctcctcta   780
ctccttctcc agggtcgggg tgcgaggtgg atgggtgtga ggtgtgcgag ggggactccg   840
ctgcgcggtg cgccaggtgc cgtgagggct actccctgac ggacgagaag acgtgcctgg   900
cgaaccacga tggcggcgtg gcggcggcgt cgagcggagc ggtggctgcg gctgctgtgt   960
gggcggctgt gctgttgagc gtggggctgg tggcgtgagg gtgcggcggc cccctcttct  1020
ctgtggtgcc cctggtgcct gccctcgccc ccagcacggc gtcgtcgctg ccctctcacc  1080
cccaccagcc gaaggggaga ccgacagcca cacgcacacg cgcacgcgcc gtcgtgcatc  1140
gcgtgtgctt tccgccgttg tggcgcctgc gcggatgcac gggcatgcgg aggcgtgcat  1200
gcgtgtgcgc gtgccagctc ttgtgtgtct ctccgtgtgg ccagcagtcg gcacccgcgc  1260
cgatcgaatg tgcgcgcggc ggcggtgtgt cgccttggac agcggatgcg gcgcccgccc  1320
ctcgccgtgt gccctgcggt ctgctgtgct gccgcgcgag cgacgtacgg a           1371
```

<210> 4
<211> 1203
<212> DNA
<213> Artificial sequence

<220>
<223> Leishmania amazonensis promastigotes surface antigens

<400> 4

```
tcggcgtgtg gatgggcgat gtggattata ccggcacgct gccggagatg cctgcgagcg    60
tcgactacaa ggacgtcatg atcacggaac tgaacttcgg cgcaatgggc cagggactga   120
gcgggacgct gcccccctca tggagctcga tgaagcagct gatcgatctg gatctggagg   180
gcactaaggt gtccggcacg ctgccgcccg agtggagtgg gatggcgaag gccgaggccc   240
tgcagctgaa gtactgcgat ctgtccggga gtctgccccc ctcgtggtct tcgatgcaga   300
agctgcgtat cgtctcactg agcggcaacc acttctgcgg gtgcgtgccc gactcgtgga   360
gggagaagga ccgcctcgat gtgaccatcg aggaatggca catgggcgag gactgcaagc   420
ttgctaacgc ctgccgcccg actgctgctc cgggaacgac cacgactaac ccgcccacca   480
```

ccaccggcac cccagcagcc tcctctactc cttctccagg gtcggggtgc gaggtggatg 540
ggtgtgaggt gtgcgagggg gactccgctg cgcggtgcgc caggtgccgt gagggctact 600
ccctgacgga cgagaagacg tgcctggcga accacgatgg cggcgtggcg gcggcgtcaa 660
gcggagcggt ggctgcggct gctgtgtggg cggctgtgct gttgagcgtg gggctggtgg 720
cgtgagggtg cggcgggccc ctcttctctg tggtgcccct ggtgcctgcc ctcgcccccg 780
gcacggcgtc gtcgctgccc tctctcaccc ccaccagccg acggggagac cgacagccac 840
acgcgcacgc gcacacgccg tcgtgcatcg cgtgtgcttt ccgccgttgt ggcgcctgca 900
cggatgcacg ggcatgcgga ggcgtgcatg cgtgtgcgcg tgccagctct tgtgtgtctc 960
tccgtgtggc cagcagtcgg caccccgcgcc gatcgaatgt gcgcgcggcg gcggtgtgtc 1020
gccttggaca gcggatgctg gcgcccgccc ctcgcgtgtg cctcggtctg cgtgtcgtgg 1080
ccgcgcgagc gacgtacgga gtgcgctgtc gccgggtggt ggccgtgcgg cgggcggggg 1140
ctcctctgtg tttctctatt tctctgttcc ctgttgacct caaaaaaaaa aaaaaaaaaa 1200
aaa 1203

<210> 5
<211> 1207
<212> DNA
<213> Articial sequence

<220>
<223> Leishmania amazonensis promastigotes surface antigens

<400> 5

ccggcgtcgg cgtgtggatg ggcgatgtgg attataccgg cacgctgccg gagatgcctg 60
cgagcgtcga ctacaaggac gtcatgatca cggaactgaa cttcagcgca atgggccagg 120
ggctgagcgg gacgctgccc ccctcatgga gctcgctgac gtccttgata tcactgtgca 180
tcgaaaagtc tgagaaggtc accggcacgc tgcctgccca gtggagctcg atgacgtcgc 240
tggacaacct taacctgcac gacacggcgg tctccggcac gctgccgccc gagtggagtg 300
ggatgacgtc gctggacgac cttaacctgc acgacacggc ggtctccggc acgctgcctg 360
cccagtggag ctcgatgaag cagctgatcg atctggatct ggagggcact aaggtgtccg 420
gcacgctgcc gcccgagtgg agtgggatgg cgaaggccga ggccctgcag ctgaagtact 480
gcgatctgtc cgggagtctg ccccccctcgt ggtcttcgat gcagaagctg cgtatcgtct 540
cactgagcgg caaccacttc tgcgggtgcg tgcccgactc gtggagggag aaggaccgcc 600
tcgatgtgac catcgaggaa tggcacatgg gcgaggactg caagcttgct aacgcctgcc 660
gcccgactgc tgctccggga acgaccacga ctaacccgcc caccaccacc ggcaccccag 720
cagcctcctc tactccttct ccagggtcgg ggtgcgaggt ggatgggtgt gaggtgtgcg 780

agggggactc cgctgcgcgg tgcgccaggt gccgtgaggg ctactcctga cggacgagaa 840
gacgtgcctg gcgaaccacg atggcggcgt ggcggcggcg tcaagcggag cggtggctgc 900
ggctgctgtg tgggcggctg tgctgttgag cgtggggctg gtggcgtgag ggtgccgccg 960
ccccctcttc tctgtggtgc ccctggtgcc tgccctcgcc cccagcacgg ggtcgtcgct 1020
gccctctcac ccccaccagc cgaaggggag accgacagcc acacgcacac gcgcacgcgc 1080
cgtcgtgcat cgcgtgtgct ttccgccgtt gtggcgcctg cgcggatgca cgggcatgcg 1140
gaggcgtgca tgcgtgtgcg cgtgccaact cttgtgtgtc tctccgtgtg gccagcagtc 1200
ggcaccc 1207

<210> 6
<211> 3088
<212> DNA
<213> Artificiel sequence

<220>
<223> Leishmania infantum promastigotes surface antigens

<400> 6

gcgctgctgc cgctggcgct gttgtgtgtg tgctggggcc gcgccacgca cacgcacggt 60
agtgaggggg agccgcagcg accgaccggg cggagcgggc gggcggaggg gggcgctccc 120
gcccgctggt catgctctct gtttcgctgg ccggcctctc tacgccgctg gcgtgggcgg 180
agctccgcgc tgcgtatcgc tcgcccctcg ctgcccctcc ctgcccctcc tcatgtgcac 240
tgctccctcc ctctccctcc ctctacactc ctcgctgtcc cctcggccga cctccacgga 300
cacgcagacg tgcgtgcgca tacacaccac ccctcacctc gctgctgctg ctgtgacagc 360
tctacggacc ctgcccagtc gctgcgcccc cgccacccgc ctctgtcccc cgcacgaggg 420
tacctacgac gtgccggcca ccccgctctg cccgataagc tgagctggcg ctcacgcccg 480
agcaatcccc tcacggatct gctgccgcgc cgcactgctc ttgaccctgg ctgcgaatgg 540
cgctgtgcgt gcgtcggctg gtgctggcgg cgaccctcgc cgctgtggtg gcgctgctgc 600
tgtgcacgag cagtgcgccg gtggcgcgtg ctgctgtgaa ggatgacttc actgctgcgc 660
agcggacgaa cacgctggcg gtgctggagg cgtttgggcg tgcgatccct gagcttggga 720
agctgtggaa gggcgacgac ttctgctttt gggagtcggt cgtgtgcgat gtgaccgaag 780
tgtacttgtg ggaaatcggt gcgacgtata ccggcacgct gccggagatg cctgtggacg 840
tcgactacac ggccgtcatg gtcaagcacc tcgacttttc ccaaatgggg ctggggctga 900
gcggaacgct gccggacagc tggagcaggc tgcagggact gacctcactt acgttgtcgg 960
gctgcggcgt gagcggtacg ctgccccct cgtggcgctc gatgaagtct ttggtgtcgt 1020
tgtggattga gagttgtgaa agtgttaccg gcaagctgcc gcctgagtgg agctcgatga 1080

aatcgctgag agatctccat ctgcatggcg cgaaggtttc cggcacgctg ccgcctgagt 1140
ggagcacgat gaaatcgctg acccttctcg atctgcagga cactcaggtt accggcagtc 1200
tgccgcctga gtggagctca atgaaatcca tgaccattct cagtctgaat ggcgcgaagg 1260
tttccggcac gctgccaccc cagtggagct cgatgacatc gctgagcctt ctcagtctgg 1320
agggtactca gctctccggc acgctaccgc cccagtggag tgggatgaca tcgctggtca 1380
cgctttttct gcagggtact caggtctccg gcactctgcc gccgcagtgg agatcgatgt 1440
tgaatgccga gttcctgcag ctggagaact gcgacctgtc cggctgtttg ccccccgagt 1500
gggctgcgat gccgaagctg cgtcatgtcg aacttaaggg caaccagttc gccgggtgtg 1560
tgccggactc gtgggctcag aaggccggtc tcgttgtgga aatcgaggat aagcacacgg 1620
gcaacagctg cattgctggt gcggactgcg caacgacgac cacgaccacc actgaaccca 1680
cgtccactgc gagcccaaca gccacgccta cctctgcccc cgagacggag tgcgaggtgg 1740
atgggtgtga ggtgtgcgat ggggactccg cggcgaggtg cgccaggtgc cgtgagggct 1800
acttcctgac ggacgagagg acgtgcctgg tgtaccgcga tggcggcgtt gtggccgtgt 1860
cgatcggagc ggctgctgcc gctgttgtgt gcatggctgt gctgctgagc gtggggctgg 1920
cggcgtgagg atgccgctgc tgtcgcgcgc aggcggcggc acccgctgcg tggcacacga 1980
ctgcgtgctt gcgtgcagca ccgcgccctg cattggcgtg cgtgtgcgcg tctgtgtgtg 2040
catggctgct gacggtgcct ttcgtcctgc ctctcgctgc ctctgcctct ctccgcgtgt 2100
gaatgctgtg ggctgtgttt ggggctctcg tgcggcgctg ctgtacggct gctgcttctt 2160
ctccaccctc ctctctcgca tgccggcgag ggaggggtgg cacgtgcgcg tgtgccgctg 2220
cgcttgcgag tgcgtctgtg tgtgggcctt caccacgtgc tacggtcacg ccttctcggc 2280
tggccactcg cggcgctgag ggcggtgtgc ccttcccctc gagcgccgtc gcactctctt 2340
ccgcgcgcct gcgcgggctt cttcgtgcgc tgtgctcagc cgtgcgctct cacctctttc 2400
ccttttcatt cgcttgtctt ctctcttctc cccccgcact gcggtctccc ctcctctgcc 2460
gtgcggtgcg caggcgggtg acttgccgtt gcgtctcccc ctttcgtgga gcgctgagcc 2520
gatccccctt cggcctccct cctccctcct cccgtgggtc ctgtctgttg tacatcgtcg 2580
gaccgtctct tcgtgttgcc tctccgcacc ttccgcaaat ctgcgctcgc ctgtgccgcc 2640
tctcggactt tatccttact gtgattgtat tctcacggtg cgtctccgtg tgtgtgtgtg 2700
ccacgcaccg cttcttccat gtgtgtcctt gcttgctctc gtctgccccc ccccctctgc 2760
ctcacacatt ccgtgcgtgt gtgcatcacc gttgggcggc gacatcggtg cccgtccctg 2820
ccaccctcta ctccctcatt ctcttgccac ttcgtgggcg gtgcgtgcat gcatggatgt 2880
atatacacgc atagaggggt ggggacgcgg gggatcctct agagtcgacc tgcaggcatg 2940
caagcttggc gtaatcatgg tcatagctgt ttcctgtgtg aaattgttat ccgctcacaa 3000
ttccacacaa catacgagcc ggaagcataa agtgtaaagc ctggggtgcc taatgagtga 3060
gctaactcac attaattgcg ttgcgctc 3088

<210> 7
<211> 18
<212> DA
<213> Artificial séquence

<220>
<223> Amorce

<400> 7
catcgcttac cccttgtt 18

<210> 8
<211> 16
<212> DNA
<213> Artificial sequence

<220>
<223> Amorce

<400> 8
gtggccaatc gcgaca 16

<210> 9
<211> 21
<212> DNA
<213> Artificial sequence

<220>
<223> Amorce

<400> 9
atggcgctgt gcgtgcgtcg g 21

<210> 10
<211> 20
<212> DNA
<213> Artificial séquence

<220>
<223> Amorce

<400> 10
gcgcgcgaca gcagcggcat 20